# EUROPÄISCHE PATENTSCHRIFT

(11) **EP 2 290 049 B1**
(45) Veröffentlichungstag und Bekanntmachung des Hinweises auf die Patenterteilung: **15.08.2012**
(21) Anmeldenummer: 10183637.7
(22) Anmeldetag: 18.08.2000
(51) Int. Cl.: C12M 3/00

(54) **Bioreaktor**
Bioreactor
Bioréacteur

(30) Priorität: 08.09.1999 EP 99810806
(43) Veröffentlichungstag der Anmeldung: 02.03.2011
(62) Teilanmeldung aus: 00810733.6
(73) Patentinhaber: Levitronix Technologies, LLC, Waltham MA 02451 (US)
(72) Erfinder: Hugel, Jörg, 8008, ZÜRICH (CH); Schoeb, Reto, 9200, Gossau (CH)
(74) Vertreter: Steiner, Peter

(56) Entgegenhaltungen:
- EP-A1- 0 197 299
- WO-A1-86/07604
- WO-A1-86/07605

## Beschreibung

Die Erfindung betrifft einen Bioreaktor gemäss dem Oberbegriff des unabhängigen Patentanspruchs. Die erfindung wird nur von den ansprüchen Limitiert.

Über Bioreaktoren spricht man heutzutage häufig, wenn man über das Gebiet des "tissue engineering" spricht. Ein herausragender Zweck dieses Gebiets ist es, biologische Substitute für geschädigtes Gewebe oder Organe herzustellen. Aber es gibt auch noch eine grosse Anzahl anderer Zielgebiete, beispielsweise kann man die Wirksamkeit oder Toxizität von Pharmazeutika an solchem Gewebe erproben, wodurch möglicherweise in der Zukunft eine grosse Anzahl von Tierversuchen und/oder klinischen Versuchen am Menschen unterbleiben kann.

EP 0197299 beschreibt einen inkubator, in dem die zellen zirkulieren.

Bei der Herstellung solcher Gewebe werden Bioreaktoren eingesetzt. Hierbei kommen unterschiedlichste Reaktortypen zum Einsatz, wie beispielsweise die sogenannten Hohlfaserreaktoren. Ein solcher Hohlfaserreaktor ist beispielsweise aus der US-A-5,622,857 bekannt. Dieser Reaktor umfasst einen Reaktionsbehälter, durch welchen ein zentraler Strang von porösen Hohlfasern verläuft, durch welchen eine Nährlösung gepumpt wird. Dieser zentrale Strang von Hohlfasern ist konzentrisch von mehreren Strängen von Hohlfasern umgeben, durch welche ein gasförmiges Medium gefördert wird. Auch die Hohlfasern dieser Stränge sind so beschaffen, dass das gasförmige Medium - beispielsweise Sauerstoff oder Kohlendioxid - zumindest teilweise aus diesen Strängen austreten bzw. in diese Stränge eintreten kann.

Sowohl zwischen dem zentralen Strang und den diesen zentralen Strang umgebenden Strängen als auch zwischen diesen den zentralen Strang umgebenen Strängen und der Behälterwand wird jeweils ein Hohlraum gebildet. Dort kann das mit den verschiedenen Medien zu beaufschlagende Gut - z.B. Stammzellen oder was auch immer - bereitgestellt werden, gegebenenfalls auf einem sogenannten "micro-carrier" oder einem bioabbaubaren Matrixmaterial. Durch die flüssige Nährlösung, welche zu einem gewissen Grad aus den Poren des zentralen Strangs austreten kann, erfolgt eine Ernährung des Guts und durch das gasförmige Medium erfolgt dessen Versorgung mit Sauerstoff.

Da in der Regel die wieder aus dem Reaktionsbehälter austretende Nährlösung rezirkuliert und wieder für den nächsten Durchlauf verwendet wird, gegebenenfalls durch weitere Nährlösung ergänzt, kann natürlich ohne weiteres eine "Kontaminierung" erfolgen. Dies ist insofern von Bedeutung, als dann für das Gewebe eines anderen Patienten diese Teile sehr intensiv sterilisiert werden müssen, damit es nicht zu einer Kontamination kommen kann. Trotz aller noch so intensiven Sterilisierung der einzelnen Teile kann jedoch nicht immer eine hundertprozentige Sterilisierung gewährleistet werden. Die Sterilität ist aber für den Erfolg des "tissue engineering" von zentraler Bedeutung.

Für die Reaktionsbehälter und für die Zuführleitungen werden bereits bei jeder Anwendung ein neuer Reaktionsbehälter bzw. neue Zuleitungen verwendet.

Aber auch die Pumpe kann bei derartigen Anwendungen kontaminiert werden. Da die Pumpen von der Aufwandsseite her betrachtet immer noch sehr aufwendigen Vorrichtungen sind, werden diese mit relativ grossem Aufwand sterilisiert.

Diesem Nachteil widmet sich die vorliegende Erfindung. Es ist eine Aufgabe der Erfindung, einen Bioreaktor vorzuschlagen, bei welchem mit grosser Sicherheit die Sterilität gewährleistet werden kann, um den Erfolg eines mit diesem Reaktor durchzuführenden "tissue engineering" - Verfahrens nicht von vorneherein durch mangelnde Sterilität zu gefährden, andererseits soll der Aufwand hierfür so gering wie möglich sein.

Diese Aufgabe wird erfindungsgemäss durch einen Bioreaktor gelöst, wie er nachstehend beschrieben ist. Weitere vorteilhafte Ausgestaltungen ergeben sich aus den Merkmalen der abhängigen Patentansprüche.

Der Bioreaktor hat einen Reaktionsbehälter für ein mit einem Medium zu beaufschlagendes Gut, und eine Pumpe, mit deren Hilfe das Medium gefördert wird, wobei die Pumpe zur Förderung des Mediums als Einmalpumpe bzw. Teile der Pumpe als Einmalteile ausgebildet sind. Dadurch entfällt die aufwändige Sterilisierung der Pumpe.

Das Medium kann durch eine Zuführleitung in den Reaktionsbehälter gefördert werden oder es kann zumindest der Rotor der Pumpe innerhalb des Reaktionsbehälters angeordnet sein.

Weiter kann die Einmalpumpe den Reaktionsbehälter umfassen, innerhalb welchem das Pumpenrad angeordnet ist, sowie einen separaten Antriebsstator, in welchen der Reaktionsbehälter mitsamt dem darin angeordneten Pumpenrad einsetzbar ist, wobei das Pumpenrad und vorzugsweise auch der Reaktionsbehälter als Einmalteil ausgebildet ist.

Bei einem vorteilhaften Ausführungsbeispiel sind die Einmalteile der Einmalpumpe aus einem Kunststoff hergestellt sind, weil solche Teile mit grosser Zuverlässigkeit wirtschaftlich hergestellt werden können, beispielsweise durch Spritzgiessverfahren.

Bei einem weiteren vorteilhaften Ausführungsbeispiel umfasst die Einmalpumpe ein Pumpengehäuse, in welchem das Pumpenrad angeordnet ist, sowie einen separaten Antriebsstator, in welchen das Pumpengehäuse mitsamt dem darin angeordneten Pumpenrad einsetzbar ist. Das Gehäuse mitsamt dem darin angeordneten Pumpenrad ist dabei als Einmalteil ausgebildet. Dieses Ausführungsbeispiel ist insofern besonders vorteilhaft, als sämtliche "kontaminierbaren Teile", nämlich das Pumpengehäuse (Innenwand) sowie das darin angeordnete Pumpenrad nach jeder Anwendung auf einfachste Art und Weise ausgewechselt werden können, und die aufwändigeren Teile (elektrische Versorgung des Antriebs, etc.) erhalten werden und für die nächste Anwendung wiederverwendet werden können, ohne dass irgendeine Gefahr der Kontaminierung besteht. Im übrigen stellt der elektrische Antrieb nicht nur von der technischen, sondern auch von der wirtschaftlichen Seite her den aufwändigsten Teil der Pumpe dar. Dieser muss aber nicht ersetzt werden, sondern nur das wenig aufwändige Pumpengehäuse mit dem darin befindlichen Pumpenrad.

Bei einer vorteilhaften Weiterbildung dieses Ausführungsbeispiels sind in dem Pumpenrad Permanentmagnete angeordnet, die dann zusammen mit dem vom Antriebsstator erzeugten elektromagnetischen Feld den Antrieb des Pumpenrads besorgen.

In vorteilhafter Weise kann die Einmalpumpe als Zahnradpumpe ausgebildet sein. Dies ist ein konstruktiv besonders einfacher Typ von Pumpe, der auch sehr wirtschaftlich in der Herstellung ist. Im übrigen weisen Zahnradpumpen nicht die Ermüdungserscheinungen auf wie etwa Schlauchquetschpumpen, die sonst häufig bei solchen Anwendungen eingesetzt werden.

Der Bioreaktor kann beispielsweise als Hohlfaserbioreaktor ausgebildet sein, wie er eingangs bereits anhand eines speziellen Ausführungsbeispiels erläutert wurde.

Der Bioreaktor kann aber auch als sogenannter Blasenreaktor ("airlift reactor") ausgebildet sein. Bei einem Blasenreaktor geht es im Prinzip darum, die Flüssigkeitsversorgung (Nährlösung) und die ebenso notwendige Versorgung mit Gasen wie z.B. Sauerstoff derart vorzunehmen, dass Blasen in der Flüssigkeit aufsteigen bzw. dort in der Schwebe gehalten werden.

Bei einem Ausführungsbeispiel eines solchen Blasenreaktors umfasst dieser einen Reaktionsbehälter, in welchem ein Hohlkörper angeordnet ist, dessen Mantel an seinem unteren Ende mit der Wand des Reaktionsbehälters verbunden ist und sich in Richtung zum oberen Ende des Reaktionsbehälters hin verjüngt, sodass er den Innenraum des Reaktionsbehälters in eine obere Kammer und eine untere Kammer unterteilt. Die obere und untere Stirnfläche des Hohlkörpers sind flüssigkeits- und gasdurchlässig ausgebildet (z.B. als Membran) und schliessen einen Hohlraum ein, in welchem das zu beaufschlagende Gut (z.B. die Zellen oder die micro-carrier mit den Zellen oder das bioabbaubare Matrixmaterial mit den Zellen) angeordnet werden kann. Je nach Art der Anwendung muss jedoch eine Membran bzw. müssen beide Membranen nicht zwingend vorhanden sein. Die Zuführleitung für das flüssige Medium mündet in die obere Kammer hinein und in der unteren Kammer ist eine Absaugeinrichtung für das flüssige Medium vorgesehen. Dadurch wird eine Flüssigkeitsströmung von oben her kommend durch den Hohlraum hindurch , in welchem das zu beaufschlagende Gut angeordnet ist, in die untere Kammer hinein erzeugt. In der unteren Kammer ist eine Zuführeinrichtung für das gasförmige Medium angeordnet. Dies bewirkt, dass die Blasen in der Flüssigkeit aufsteigen. Da die Geschwindigkeit der Flüssigkeitsströmung im oberen Bereich des Hohlraums aber schneller ist (kleinerer Durchmesser) als im unteren Bereich (grösserer Durchmesser) werden die aufsteigenden Blasen im oberen Bereich wieder von der Strömung nach abwärts mitgenommen, wo die Strömungsgeschwindigkeit der Flüssigkeitsströmung wieder geringer ist, weshalb die Blasen wieder aufzusteigen beginnen. Durch eine entsprechende Wahl der Strömungsgeschwindigkeit ist es so möglich, die Blasen in dem Hohlraum zu "konzentrieren", in welchem das zu beaufschlagende Gut angeordnet ist.

Bei einer Weiterbildung eines solchen Blasenreaktors ist der Reaktionsbehälter zylindrisch und der Hohlkörper kreiskegelstumpfförmig ausgebildet. Die Zuführleitung mündet dabei in einen vorzugsweise ringförmigen oder kreisförmig flächigen Verteiler, welcher in der oberen Kammer angeordnet ist und den Hohlkörper umgibt. Die in der unteren Kammer angeordnete Absaugseinrichtung für das flüssige Medium ist ebenfalls vorzugsweise ringförmig oder kreisförmig flächig ausgebildet. Dies ergibt einerseits eine gut kontrollierbare Strömung und gewährleistet andererseits, dass der Verteiler und auch die Absaugeinrichtung auch dann verwendet werden können, wenn die Abmessungen des Behälters und des darin angeordneten Hohlkörpers einmal nicht so genau den gewünschten Abmessungen entsprechen sollten.

Bei einem weiteren Ausführungsbeispiel des Blasenreaktors umfasst dieser einen Reaktionsbehälter, in welchem ein Hohlkörper angeordnet ist, dessen Mantel an seinem oberen Ende mit der Wand des Reaktionsbehälters verbunden ist und sich in Richtung zum unteren Ende des Reaktionsbehälters hin verjüngt, sodass er den Innenraum des Reaktionsbehälters in einen obere Kammer und eine untere Kammer unterteilt. Die obere und untere Stirnfläche des Hohlkörpers sind jeweils flüssigkeits- und gasdurchlässig ausgebildet (z.B. als Membran oder als Netz oder als Filtermatte) und schliessen einen Hohlraum ein, in welchem das zu beaufschlagende Gut angeordnet werden kann. Die Zuführleitung für das Medium mündet hier in die untere Kammer hinein, wobei dem flüssigen Medium bereits das gasförmige Medium beigemischt ist (z.B. mittels eines Oxygenators). In der oberen Kammer ist eine Absaugeinrichtung für das Medium vorgesehen, wodurch eine gewünschte Strömungsgeschwindigkeit erzeugt werden kann. Die Strömungsgeschwindigkeit ist nach oben gerichtet, wobei sie im unteren Bereich des Hohlkörpers am grössten ist und durch die Aufweitung des Hohlkörpers nach oben hin abnimmt. Dadurch werden die Zellen, die sich zwischen den beiden Membranen befinden, in der Schwebe gehalten, wodurch sich gute Bedingungen für das Wachstum eines dreidimensionalen Zellverbunds (Gewebe) einstellen.

Bei einem anderen Ausführungsbeispiel kann der Reaktionsbehälter einen flexiblen Beutel umfassen, welcher in eine formstabile Aufnahme einsetzbar ist. Diese Aufnahme kann beispielsweise als Wärmemantel ausgebildet sein und die Temperatur des Mediums auf einer gewünschten Temperatur halten. Ausserdem verleiht der Wärmemantel dem flexiblen Beutel die erforderliche Stabilität.

Schliesslich kann es bei allen Ausführungsbeispielen so sein, dass nicht nur die Pumpe oder Teile davon als Einmalteile ausgebildet sind, sonder es kann so sein, dass ausser der Pumpe bzw. den Teilen der Pumpe auch sämtliche anderen Bestandteile des Bioreaktors, die mit dem Medium in Kontakt kommen, als Einmalteile ausgebildet sind. Der Bioreaktor kann damit als assemblierter Bioreaktor bereits steril verpackt geliefert werden und wird nach jeder Anwendung ersetzt.

Der wesentliche Erfindungsgedanke ist also die Verwendung einer Einmalpumpe bzw. einer Pumpe mit als Einmalteilen ausgebildeten Teilen in einem Bioreaktor, was jedoch, wie oben erläutert, auf unterschiedlichste Weise realisiert werden kann. Jedoch kann in vorteilhafter Weise auch ein Bioreaktor verwendet werden, bei dem sämtliche Teile, die mit dem Medium in Kontakt kommen, als Einmalteile ausgebildet sind. Auf diese Weise wird eine Kontamination bei einer zweiten Anwendung (die es dann mit demselben Bioreaktor eben nicht gibt) zuverlässig vermieden.

Das künstliche Herstellen von Gewebematerial, im Englischen als "Tissue Engineering" bezeichnet, gewinnt zunehmend an Bedeutung, um biologische Substitute für geschädigtes Gewebe oder geschädigte Organe herzustellen. Künstliches Gewebematerial ist herstellbar, indem Zellkulturen in vitro an oder in einem Gewebeträger, auch als Matrix bezeichnet, eingelagert werden. Der Gewebeträger besteht beispielsweise aus einem synthetischem Polymer oder aus einem biologischen Material wie Kollagen. Ein derartiger Gewebeträger wird auch als "Scaffold" bezeichnet. Die Zellen werden auf dem Gewebeträger ausgesät und beginnen sich, sofern die Umgebungsparameter physiologisch angepasst sind, zu vermehren. Der Gewebeträger kann derart ausgestaltet sein, dass dieser sich mit der Zeit auflöst, sodass nach einer bestimmten Zeit nur noch das aus dem Zellen gebildete Gewebeteil vorhanden ist. Der Gewebeträger und/oder das darauf gebildete Gewebeteil wird nachfolgend als "Gut" bezeichnet. Die für das Zellwachstum erforderlichen Bedingungen werden in einem Bioreaktor erzeugt, innerhalb welchem dem Gut der erforderliche Sauerstoff sowie ein Nährmedium zugeführt wird, und innerhalb welchem das Gut während mehreren Tagen bis Wochen verbleibt, bis die gewünscht Grösse erreicht ist. Die geometrische Form, welche das künstlich erzeugte Gewebematerial während dem Wachstum annimmt, wird wesentlich beeinflusst durch die Massnahmen, mit welchen das Gut im Bioreaktor gehalten wird.

Nachfolgend wird also unter dem Begriff "Gut" sowohl der Gewebeträger an sich, als auch der Gewebeträger mit angelagerten Zellen verstanden, oder falls der Gewebeträger abbaubar ausgestaltet ist, die künstlich erzeugte Zellkultur bzw. das künstlich erzeugte Gewebeteil.

Als Verfahren zum schwebenden Halten eines Gutes in einem Bioreaktor, wird vorzugsweise das Gut mit einem Fluid beaufschlag, wobei die Strömung des Fluides derart der Schwerkraft entgegengesetzt wirkt, dass das Gut in der Schwebe gehalten wird.

Dieses Verfahren weist den Vorteil auf, dass das Gut berührungslos im Bioreaktor gehalten wird, indem das Fluid, üblicherweise eine Flüssigkeit, eine derartig ausgebildete Strömung aufweist, dass das Gut von der entgegengesetzt der Schwerkraft wirkenden Strömung berührungslos gehalten wird. Dabei wird das Gut üblicherweise auch ständig in Bewegung gehalten, sodass sich dessen Lage ständig verändert. Dieses Verfahren weist den Vorteil auf, dass die Zellen gleichmässig am bzw. im Gut wachsen und das Wachstum des Guts begünstigt wird. Nachteilig an den bisher bekannten Verfahren zum künstlichen Herstellen von Gewebe ist die Tatsache, dass nur flache, im wesentlichen zweidimensionale Gebilde herstellbar waren.

In einem besonders vorteilhaft ausgestalteten Verfahren weist das Fluid in zur Schwerkraft entgegengesetzter Richtung eine zunehmend geringere Strömungsgeschwindigkeit auf. Dieses Strömungsverhalten wird beispielsweise dadurch erzeugt, dass das strömende Fluid von unten in einen sich gegen oben erweiternden, kegelstumpfförmigen Hohlkörper geleitet wird. Der sich gegen oben erweiternde Querschnitt des Hohlkörpers bewirkt, dass sich die Strömungsgeschwindigkeit im Hohlkörper mit zunehmender Höhe reduziert. Das Gut wird im Innenraum des Hohlkörpers ständig in der Schwebe gehalten, wobei die Seitenwände des Hohlkörpers eine seitliche Bewegung des Guts begrenzen, sodass sich das Gut ständig in der aufwärts strömenden Flüssigkeit befindet. Mit zunehmendem Zellwachstum erhöht sich das Gewicht des Guts, sodass das Gut im Innenraum des Hohlkörpers sich leicht nach unten bewegt und dort wieder eine neue Gleichgewichtslage finde. Das Gut sucht somit selbstständig die jeweilige Gleichgewichtslage. Es kann sich jedoch als vorteilhaft erweisen die Lage des Gutes mit einem Sensor zu überwachen und mit Hilfe des gemessenen Signals die Geschwindigkeit des aufströmenden Fluides zu beeinflussen. So kann die Geschwindigkeit des Fluides beispielsweise derart geregelt werden, dass das Gut ständig in einer vorbestimmten Lage in der Schwebe gehalten wird.

In einem vorteilhaften Verfahren wird nebst der Aufwärtsströmung innerhalb des Bioreaktors zudem eine Abwärtsströmung erzeugt, wobei dem abwärtsströmenden Fluid, üblicherweise eine Flüssigkeit, ein gasförmiges Fluid wie Luft oder Sauerstoff zugeleitet wird. Die Geschwindigkeit des abwärts strömenden Fluides wird vorteilhafterweise derart gewählt, dass das eingeleitete, gasförmige Fluid verlangsam oder gar nicht mehr aufsteigt, sodass das gasförmige Fluid relativ lange im strömenden Fluid verbleit und von diesem aufgenommen bzw. absorbiert werden kann.

Ein vorteilhaft ausgestalteter Bioreaktor umfasst einen Behälter für ein mit einem Fluid zu beaufschlagendes Gut, wobei der Behälter einen ersten Strömungsraum umfasst, welchem ein strömendes Fluid zuführbar ist, und wobei der erste Strömungsraum derart ausgestaltet ist, dass das darin von unten nach oben strömende Fluid mit zunehmender Höhe eine kleinere Geschwindigkeit aufweist. In einer besonders vorteilhaften Ausgestaltung weist der Strömungsraum einen sich gegen oben erweiternden Querschnitt auf.

In einer weiteren vorteilhaften Ausgestaltung ist innerhalb des Bioreaktors ein Flussleitmittel angeordnet, welches einen sich von unten nach oben erweiternden Strömungsraum ausbildet. Vorzugsweise bildet dieses Flussleitmittel innerhalb des Bioreaktors zudem einen weiteren, sich von oben nach unten erweiternden zweiten Strömungsraum aus, in welchen ein gasförmiges Fluid einleitbar ist.

In einer weiteren, vorteilhaften Ausgestaltung ist innerhalb des Bioreaktors ein antreibbares Pumpenrad angeordnet, mit dessen Hilfe innerhalb des Bioreaktors die Strömung des Fluides erzeugbar ist. Vorteilhafterweise ist das Pumpenrad magnetisch an einen ausserhalb des Gehäuses des Bioreaktors angeordneten Antrieb gekoppelt. Das Bioreaktorgehäuse sowie das Pumpenrad sind vorteilhafterweise als Einweg- bzw. als Einmalprodukt konzipiert, sodass diese nach einer einmaligen Verwendung entsorgt werden. Diese Teile sind kostengünstig herstellbar. Beispielsweise umfass das Pumpenrad ein Flügelrad aus Kunststoff, in welchem ein Permanentmagnet eingegossen ist. Alle kostspieligen Komponenten wie die Antriebsvorrichtung sind ausserhalb des Bioreaktors angeordnet. Die Ausgestaltung des Bioreaktors als Einmalprodukt weist den Vorteil auf, dass kein aufwendiger Reinigungsprozess erforderlich ist, und dass eine Kontamination der künstlich hergestellten Gewebematerials weitgehend ausgeschlossen ist. Das Vermeiden einer Kontamination ist von entscheidender Bedeutung, da das Gut beispielsweise 4 bis 8 Wochen im Bioreaktor verbleibt, bis genügend künstliches Gewebematerial gebildet ist. Da der Bioreaktor über kein Immunsystem verfügt, können bereits kleinste Verunreinigungen wie Bakterien, Pilze oder Viren zur Folge haben, dass das hergestellte künstliche Gewebe abstirbt oder kontaminiert wird. Durch die Ausgestaltung des Bioreaktors als Einmalprodukt ist künstliches Gewebematerial kostengünstig und zuverlässig herstellbar.

Im folgenden wird die Erfindung anhand von Zeichnungen näher erläutert. Es zeigen in schematischer Darstellung und/oder im Schnitt:
- Fig. 1: eine Darstellung eines Ausführungsbeispiels eines erfindungsgemässen Bioreaktors, der als Hohlfaserreaktor ausgebildet ist;
- Fig. 2: ein weiteres Ausführungsbeispiel eines erfindungsgemässen Bioreaktors, der als Hohlfaserreaktor ausgebildet ist;
- Fig. 3: ein Ausführungsbeispiel eines erfindungsgemässen Bioreaktors, der als Blasenreaktor ausgebildet ist;
- Fig. 4: einen Ausschnitt aus dem Blasenreaktor aus Fig. 3, vergrössert und mit zusätzlichen Details;
- Fig. 5: ein weiteres Ausführungsbeispiel eines als Blasenreaktor ausgebildeten Bioreaktors;
- Fig. 6: ein Ausführungsbeispiel einer Einmalpumpe in Form einer Zahnradpumpe, welche in einem Bioreaktor eingesetzt werden kann;
- Fig. 7: den Stator der Einmalpumpe aus Fig. 6 in einer Ansicht von oben;
- Fig. 8: die Zahnradpumpe aus Fig. 6 in einer Ansicht gemäss der Linie VIII-VIII in Fig. 6;
- Fig. 9: ein weiteres Ausführungsbeispiel eines erfindungsgemässen Bioreaktors, welcher mit einem flexiblen Beutel versehen ist;
- Fig.10: ein Ausführungsbeispiel, wie der Einlass in den Reaktionsbehälter ausgebildet sein kann, um einen Oxygenator zu ersetzen;
- Fig. 11: einen Längsschnitt durch einen ersten Bioreaktor;
- Fig. 11a: eine perspektivische Detailansicht des Flussleitmittels;
- Fig. 12a, 12b: Längsschnitte durch weitere Ausführungsbeispiele von Bioreaktoren;
- Fig. 13a -13d: Längsschnitte durch weitere Ausführungsbeispiele von Bioreaktoren;
- Fig. 14: einen Längsschnitt durch einen weiteren Bioreaktor mit einem magnetisch gekoppelten Flügelrad entlang der Linie B-B;
- Fig. 15: einen Schnitt durch Figur 14 entlang der Linie A-A;
- Fig. 16: einen Längsschnitt durch einen weiteren Bioreaktor mit einem an der verschliessbaren Öffnung angeordneten Flügelrad;
- Fig. 17: einen Längsschnitt durch einen weiteren Bioreaktor mit einem magnetisch gelagerten Flügelrad entlang der Linie D-D;
- Fig. 18: einen Schnitt durch Figur 17 entlang der Linie C-C;
- Fig. 19: einen Längsschnitt durch einen weiteren Bioreaktor.

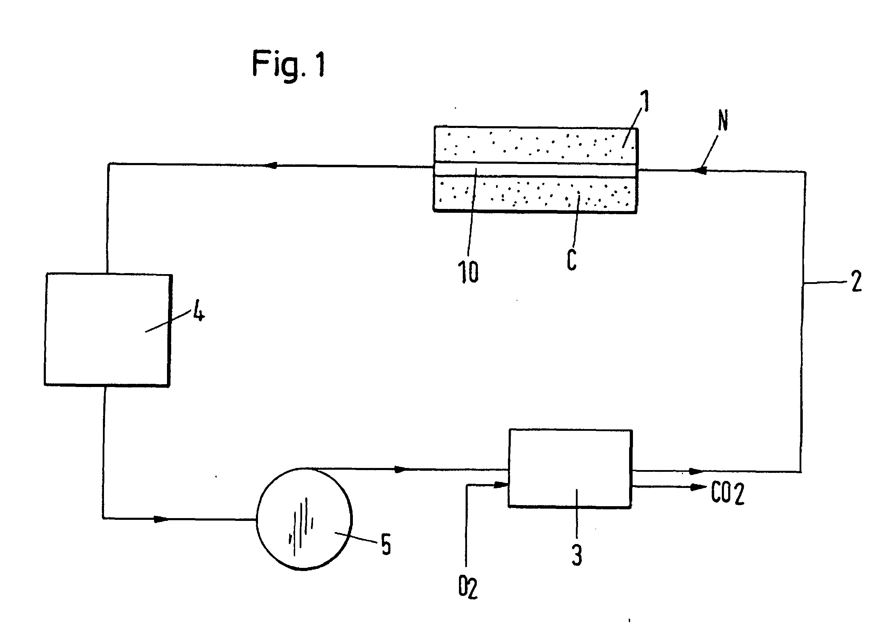

In Fig. 1 ist ein erstes Ausführungsbeispiel eines erfindungsgemässen Bioreaktors dargestellt. Man erkennt einen Reaktionsbehälter 1, welcher hier als Hohlfaserreaktor ausgebildet ist. Im Innenraum des Reaktionsbehälters sind also Hohlfasern bzw. ist ein Bündel 10 von Hohlfasern angeordnet, welches von einem flüssigen Medium, beispielsweise einer Nährlösung N, durchströmt wird, die durch eine Zuführleitung 2 zugeführt wird. Im Reaktionsbehälter 1 können Zellen C angeordnet sein, welche mit Hilfe der Nährlösung N mit Nährstoffen versorgt werden können. In der Nährlösung N kann ein Gas, beispielsweise Sauerstoff, enthalten sein, welches der Nährlösung N in einem Oxygenator 3 zugesetzt wurde, und welches ebenfalls der Versorgung der Zellen C dienen kann. Die Hohlfasern des Hohlfaserbündels 10 sind porös, sodass das Gas und die Nährstoffe bzw. Nährlösung im Reaktionsbehälter 1 die Zellen C erreichen können.

Die aus dem Reaktionsbehälter 1 abfliessende Nährlösung N kann durch ein Reservoir 4 fliessen, wo sie erneuert bzw. wieder mit Nährstoffen angereichert wird. Die Nährlösung N wird mit Hilfe einer Pumpe 5 aus dem Reservoir durch den Oxygenator 3 gepumpt, wo der Nährlösung z.B. Sauerstoff zugesetzt oder Kohlendioxid entzogen wird. Diese Pumpe 5 ist nun erfindungsgemäss als Einmalpumpe oder Wegwerfpumpe ausgebildet. Sie ist vorzugsweise aus einem Kunststoff wie zum Beispiel Polycarbonat oder Polyvinylchlorid (PVC) hergestellt, und ist beispielsweise im Spritzgiessverfahren herstellbar. Diese Einmalpumpe oder Wegwerfpumpe 5 bzw. die "kontaminierten" Teile davon werden nach jedem Einsatz ersetzt, sodass keine Kontaminationsgefahr für den nachfolgenden Einsatz auch nur bestehen kann. Die Einmalpumpe 5 bzw. diejenigen Teile davon, die als Einmalteile ausgebildet sind und ersetzt werden, werden steril verpackt angeliefert und sind vorzugsweise derart gekennzeichnet, dass sie dem Benutzer anzeigen, dass die Pumpe bzw. die Teile davon nur für den einmaligen Gebrauch gedacht sind. Die Einmalpumpe 5 kann dabei in vorteilhafter Weise als Zahnradpumpe ausgebildet sein, wie im folgenden noch genauer beschrieben wird. Diese Zahnradpumpen sind konstruktiv und damit auch herstellungstechnisch wenig aufwändig und weisen darüberhinaus die nachteiligen Ermüdungserscheinungen von Schlauchquetschpumpen, die sonst häufig bei derartigen Anwendungen zum Einsatz kommen, nicht auf.

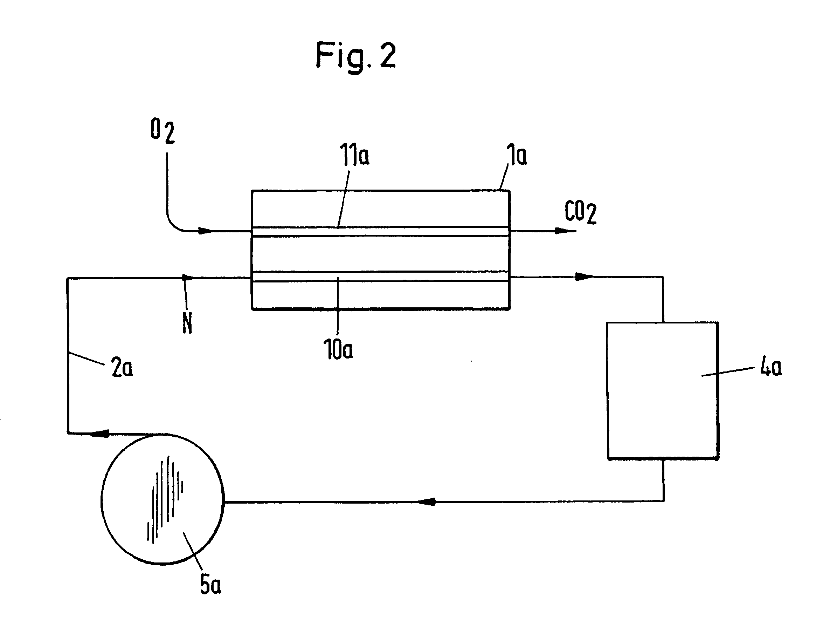

In Fig. 2 ist ein weiteres Ausführungsbeispiel eines Hohlfaserreaktors dargestellt. Der Reaktionsbehälter 1 a weist bei diesem Ausführungsbeispiel in seinem Innenraum zwei Hohlfaserbündel 10a und 11a auf, wobei das Hohlfaserbündel 10a die gleiche Funktion hat wie bei dem Ausführungsbeispiel gemäss Fig. 1. Dieses Bündel 10a wird nämlich von Nährlösung N durchströmt, welche die im Reaktionsbehälter 1a befindlichen Zellen C mit Nährstoffen versorgt. Die Zufuhr von Sauerstoff oder die Abfuhr von Kohlendioxid erfolgt bei diesem Ausführungsbeispiel aber - abweichend von dem Ausführungsbeispiel der Fig. 1 - durch das Hohlfaserbündel 11 a, welches ebenfalls im Reaktionsbehälter 1a angeordnet ist. Die Versorgung der Zellen C mit Gasen wie z.B. Sauerstoff erfolgt hier also losgelöst und separat von der Nährstoffversorgung. Die aus dem Reaktionsbehälter 1a ausströmende Nährlösung N durchströmt wieder ein Reservoir 4a, in welchem die Nährlösung erneuert bzw. wieder mit Nährstoffen angereichert werden kann, bevor sie erneut dem Reaktionsbehälter zugeführt wird. Das Pumpen der Nährlösung wird mit Hilfe der Einmalpumpe bzw. Wegwerfpumpe 5a bewirkt, die völlig analog zu der bereits anhand von Fig. 1 kurz erläuterten Pumpe ausgebildet ist. Sie kann aber beispielsweise auch als Zentrifugalpumpe ausgebildet sein.

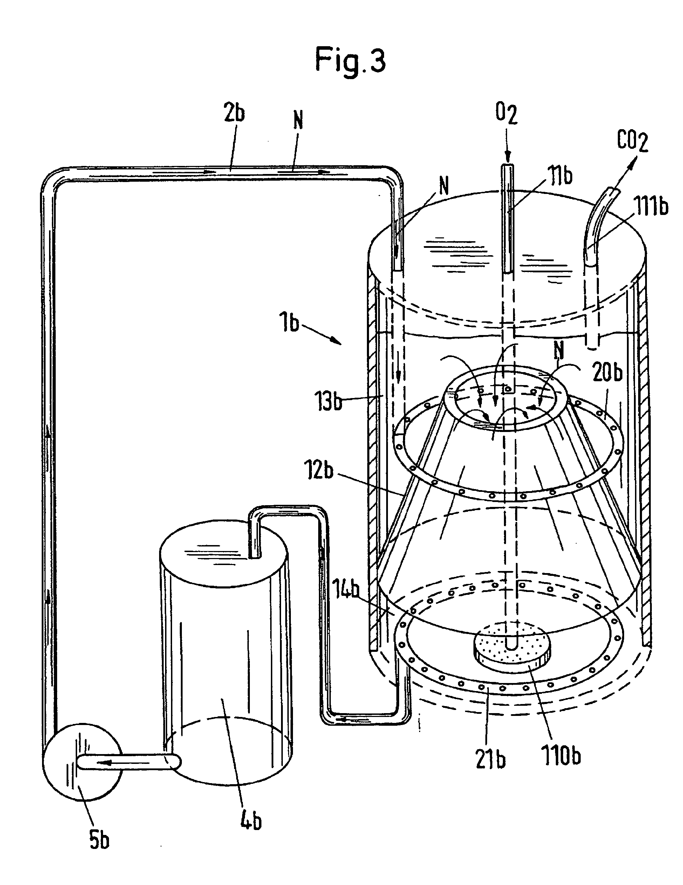

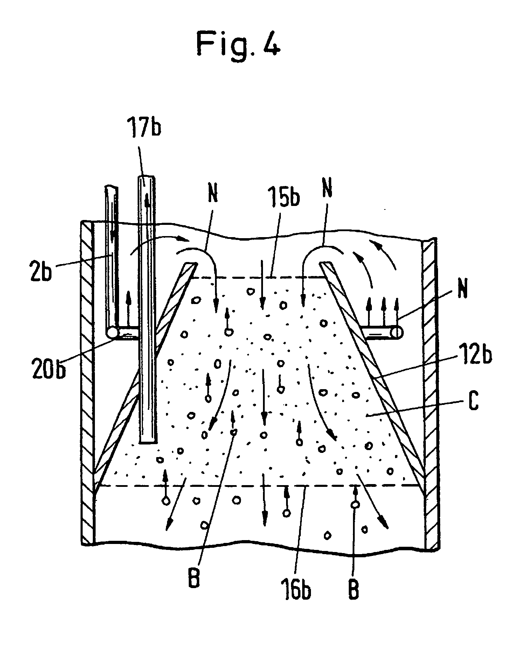

In Fig. 3 ist ein weiteres Ausführungsbeispiel eines Bioreaktors dargestellt, welcher als Blasenreaktor ausgebildet ist. Man erkennt den Reaktionsbehälter 1 b, in welchem ein kreiskegelstumpfförmiger Hohlkörper 12b angeordnet ist. An seinem unteren Ende ist der Hohlkörper 12b mit der Wand des Reaktionsbehälters 1 b verbunden. Von dort aus verjüngt sich der Hohlkörper 12b nach oben hin, sodass er den Innenraum des Reaktionsbehälters in eine obere Kammer 13b und eine untere Kammer 14b unterteilt. Die obere und untere Stirnfläche des Hohlkörpers 12b sind gasund flüssigkeitsdurchlässig ausgebildet, was man in Fig. 3 nicht erkennen kann, jedoch in Fig. 4. Dort ist ersichtlich, dass im Bereich der oberen und unteren Stirnfläche jeweils eine Membran 15b bzw. 16b angeordnet ist, welche flüssigkeits- und gasdurchlässig ist. Somit schliessen die beiden Membranen einen Hohlraum ein, in welchem die Zellen C angeordnet sind, für die jedoch die Membranen undurchlässig sind. Je nach Anwendung kann jedoch eine Membran oder auch beide Membranen nicht vorhanden sein.

Ferner erkennt man noch die Zuführleitung 2b für die Nährlösung N, welche zu einem ringförmigen Verteiler 20b führt, der in der oberen Kammer 13b angeordnet ist und den Hohlkörper 12b umgibt. In der unteren Kammer 14b ist eine ebenfalls ringförmige ausgebildete Absaugeinrichtung 21 b vorgesehen, welche mit einer Abführleitung verbunden ist, die zu dem Reservoir 4b führt. Dort wird die Nährlösung N erneuert bzw. mit Nährstoffen angereichert. Für die Förderung der Nährlösung ist die Einmalpumpe 5b besorgt, welche bereits erwähnt wurde. Die Nährlösung N wird also mit Hilfe der Einmalpumpe 5b aus dem Reservoir 4b durch die Zuführleitung 2b in den ringförmigen Verteiler 20b geführt, von dort strömt sie durch die Membran 15b und durch den Hohlraum des Hohlkörpers 12b hindurch. Sodann strömt die Nährlösung durch die Membran 16b hindurch und wird von der Absaugeinrichtung 21 b abgesaugt und zurück in das Reservoir 4b gefördert, wodurch der Nährlösungskreislauf geschlossen ist.

Was die Versorgung der Zellen C mit Gasen betrifft, z.B. mit Sauerstoff, so werden diese durch die Leitung 11b zu einem Verteiler 110b geführt, der in der unteren Kammer 14b angeordnet ist. Dort treten die Gase in Form von Blasen aus dem Verteiler 110b aus und steigen nach oben. Diesem Aufsteigen der Blasen wirkt die Flüssigkeitsströmung, die ja nach unten gerichtet ist, entgegen. Durch geeignete Wahl der Strömungsparameter kann man erreichen, dass die Blasen eine ausreichend hohe Verweilzeit in dem Hohlraum aufweisen, wo die Zellen C angeordnet sind (und der vom Hohlkörper 12b umschlossen wird). Für die austretenden Gase ist eine entsprechende Abführleitung 111b vorgesehen.

Sind nun die gewünschten Zellen C oder z.B. ein Gewebestück entsprechend gereift und man möchte Zellen C oder ein Gewebestück entnehmen, so kann dies durch einen sogenannten "Harvesting"-Kanal (Entnahmekanal) 17b erfolgen, wie dies in Fig. 4 angedeutet ist. Dort erkennt man auch besonders gut die Richtung der Strömung der Nährlösung N, die entgegen dieser Strömung verlaufende Richtung der aufsteigenden Gasblasen B, sowie die von dem Hohlkörper 12b und den Membranen 15b und 16b eingeschlossenen Zellen C.

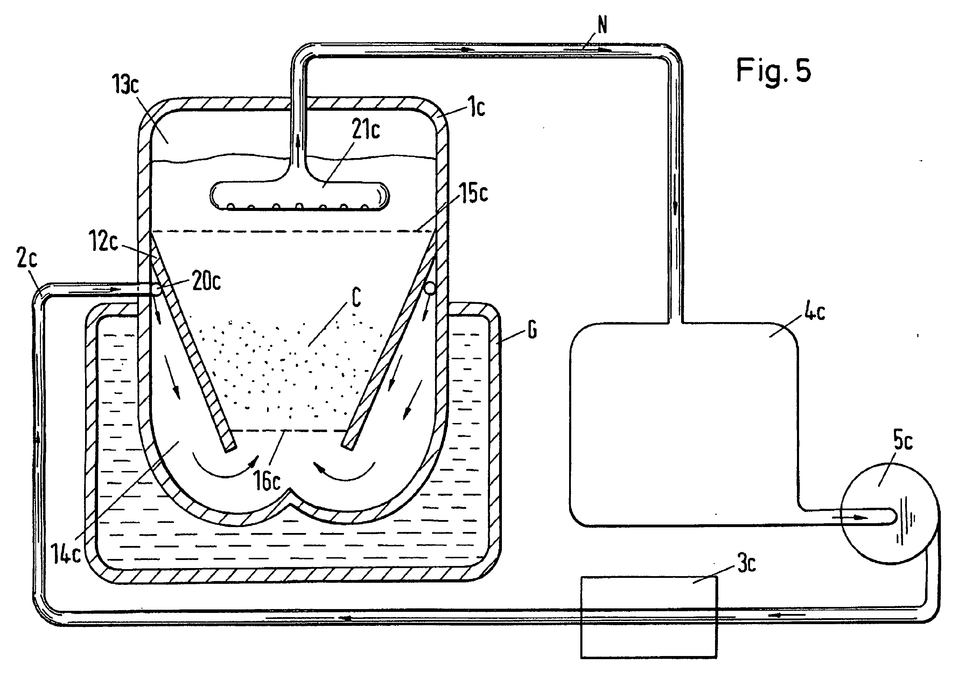

In Fig. 5 ist ein weiteres Ausführungsbeispiel eines Bioreaktors dargestellt, in welchem Zellen gezüchtet werden. Man erkennt den Reaktionsbehälter 1c, der hier von einem weiteren Gefäss G umgeben ist, in welchem beispielsweise Wasser enthalten sein kann, um beispielsweise den Reaktionsbehälter 1c auf einer gewünschten Temperatur halten zu können. In dem Reaktionsbehälter ist ein kreiskegelstumpfförmiger Hohlkörper 12c angeordnet, der den Behälter 1c in eine obere Kammer 13c und eine untere Kammer 14c unterteilt. Der Mantel des kreiskegelstumpfförmigen Hohlkörpers 12c ist an seinem oberen Ende mit der Wand des Reaktionsbehälters 1c verbunden und verjüngt sich zum unteren Ende des Reaktionsbehälters hin. Die obere und untere Stirnfläche des Hohlkörpers 12c sind gas- und flüssigkeitsdurchlässig ausgebildet, und zwar derart, dass im Bereich der oberen und unteren Stirnfläche jeweils eine Membran 15c bzw. 16c angeordnet ist, welche gas- und flüssigkeitsdurchlässig ausgebildet ist. In dem Hohlraum, der zwischen den Membranen 15c und 16c eingeschlossen wird, können Zellträger, beispielsweise aus Kunststoff oder Keramik, mit Zellen C angeordnet sein, für die die Membranen 15c und 16c undurchlässig sind. Die Zuführleitung 2c für die Nährlösung N mündet in der unteren Kammer 14c in einen ringförmigen Verteiler 20c, welcher den Hohlkörper 12c umgibt. In der oberen Kammer 13c ist eine Absaugeinrichtung 21 c vorgesehen, welche mit einer Abführleitung verbunden ist, welche zu dem Reservoir 4c führt, wo die abgeführte Nährlösung N erneuert bzw. mit Nährstoffen angereichert werden kann. Zur Förderung der Nährlösung N ist eine Einmalpumpe 5c bzw. eine Pumpe mit Einmalteilen vorgesehen, welche - wie bereits zuvor erwähnt - beispielsweise als Zahnradpumpe oder als Zentrifugalpumpe ausgebildet sein kann.

Die von der Pumpe 5c aus dem Reservoir 4c geförderte Nährlösung N gelangt in einen Oxygenator 3c, wo der Nährlösung N ein Gas wie beispielsweise Sauerstoff beigemischt oder Kohlendioxid entzogen werden kann. Die so mit Sauerstoff versetzte bzw. von Kohlendioxid befreite Nährlösung N gelangt dann im weiteren Verlauf in den ringförmigen Verteiler 20c, der in der unteren Kammer 13c angeordnet ist. Mit Hilfe der Einmalpumpe 5c und der Absaugeinrichtung 21 c wird eine Flüssigkeitsströmung erzeugt, welche durch die Pfeile in Fig. 5 angedeutet ist. Im Bereich der Membran 16c ist die Strömungsgeschwindigkeit vergleichsweise hoch - sie nimmt dann aufgrund des kegelstumpfartig sich erweiternden Hohlkörpers 12c ab. Durch eine geeignete Wahl der Strömungsparameter kann erreicht werden, dass die Zellen C im Bereich zwischen den Membranen 16c und 15c in der Schwebe gehalten werden. Dies kann die Formation eines dreidimensionalen Zellverbunds (Gewebe) begünstigen. Bei diesem Ausführungsbeispiel erfolgt die Zuführung von Nährlösung N einerseits und von Gasen wie z.B. Sauerstoff andererseits nicht getrennt, sondern die Nährlösung N wird mit Sauerstoff versetzt, bevor sie mit Hilfe der Zuführleitung 2c und dem Verteiler 20c in den Behälter 1c eingebracht wird.

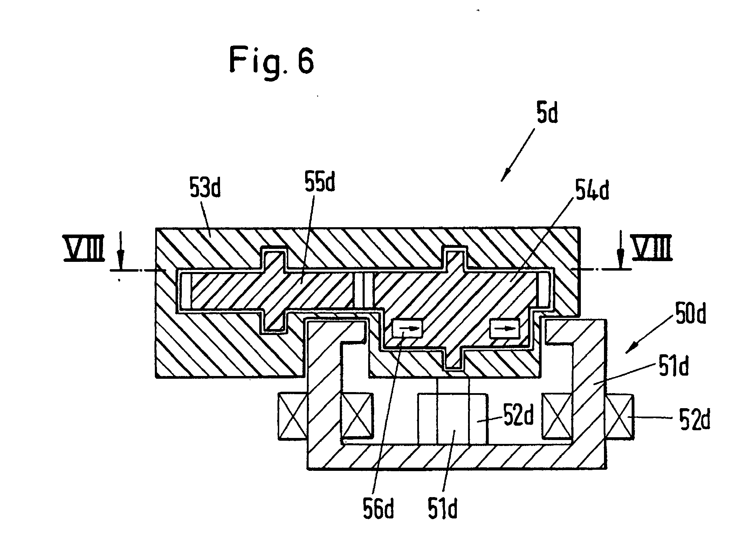

In Fig. 6 ist ein Ausführungsbeispiel für die bereits zuvor angesprochene Einmalpumpe gezeigt. Dieses Ausführungsbeispiel der Einmalpumpe ist als Zahnradpumpe 5d ausgebildet. Man erkennt den Stator 50d mit den Statorschenkeln 51 d (drei Statorschenkel sind in Fig. 6 zu erkennen), um welche herum die Antriebswicklungen 52d gewickelt sind. Ferner erkennt man ein Pumpengehäuse 53d, welches aus einem spritzbaren Kunststoff, beispielsweise aus Polycarbonat oder Polyvinylchlorid (PVC), hergestellt ist. Gleiches gilt für die beiden in dem Pumpengehäuse 53d angeordneten Zahnräder 54d und 55d, wobei in dem Antriebszahnrad 54d Permanentmagnete 56d eingespritzt sein können. Sämtliche Teile, die im Pumpengehäuse 53d angeordnet sind, also beide Zahnräder 54d und 55d, sowie das Pumpengehäuse 53d selbst, sind also hier aus einem kostengünstigen und herstellungstechnisch einfach zu beherrschenden spritzbaren Kunststoff hergestellt. Das hat zur Konsequenz, dass es ohne weiteres vertretbar ist, das Pumpengehäuse 53d mit den darin befindlichen Zahnrädern 54d und 55d nach jeder Anwendung zu ersetzen. Eine aufwändige und obendrein nicht absolut zuverlässige Sterilisation der Pumpe 5d, welche die Gefahr einer Kontamination birgt, kann dadurch vermieden werden.

Ebenfalls ist aus Fig. 6 ohne weiteres ersichtlich, dass der Aufwand zum Ersetzen des Pumpengehäuses 53d ausserordentlich gering ist. Es muss zu diesem Zweck lediglich das Pumpengehäuse aus dem Stator 50d nach oben herausgenommen werden und ein neues (steriles und daher nicht kontaminiertes) Pumpengehäuse 53d anschliessend von oben her wieder eingesetzt werden.

Eine Aufsicht von oben auf einen solchen Stator 50d mit seinen Statorschenkeln 51d sowie den um diese Statorschenkel 51d herum gewickelten Antriebswicklungen 52d ist aus Fig. 6 ersichtlich. Diese Aufsicht verdeutlicht noch einmal, dass das Ersetzen des Pumpengehäuses praktisch ohne Aufwand schnell und zuverlässig möglich ist.

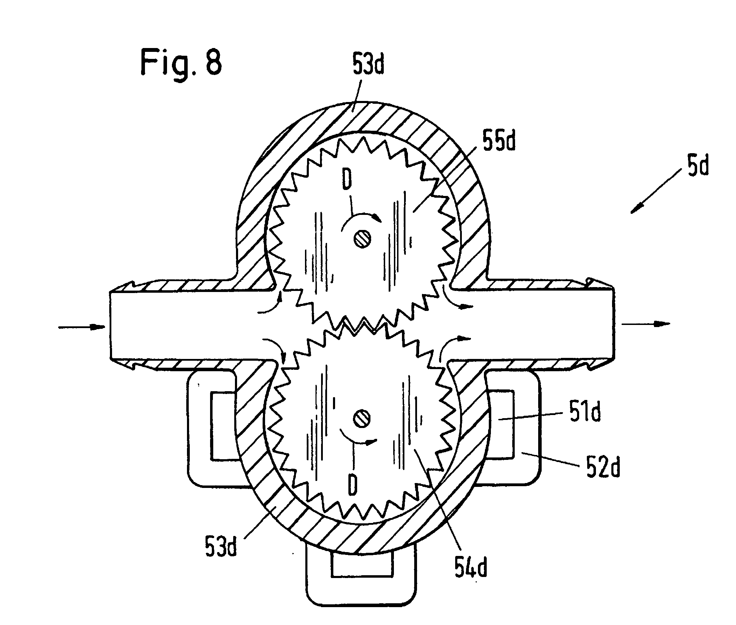

In Fig. 8 erkennt man eine Ansicht der Zahnradpumpe 5d gemäss der Linie VIII-VIII in Fig. 6, aus der das Funktionsprinzip der Zahnradpumpe ersichtlich wird. Die Drehrichtung der Zahnräder ist durch die Pfeile D angedeutet, wobei nur das Zahnrad 54d motorisch angetrieben wird und durch Eingreifen der Zähne der Zahnräder 54d und 55d ineinander das Zahnrad 55d angetrieben wird. Die Nährlösung N wird so gefördert, wie dies durch die entsprechenden Pfeile in Fig. 8 zu erkennen ist. Dadurch, dass praktisch nur ein sehr geringer Hohlraum in dem Bereich entsteht, in welchem die Zähne der beiden Zahnräder 54d und 55d ineinandergreifen, wird die Nährlösung praktisch zwischen dem jeweiligen Zahnrad 54d bzw. 55d und dem Pumpengehäuse 53d gefördert.

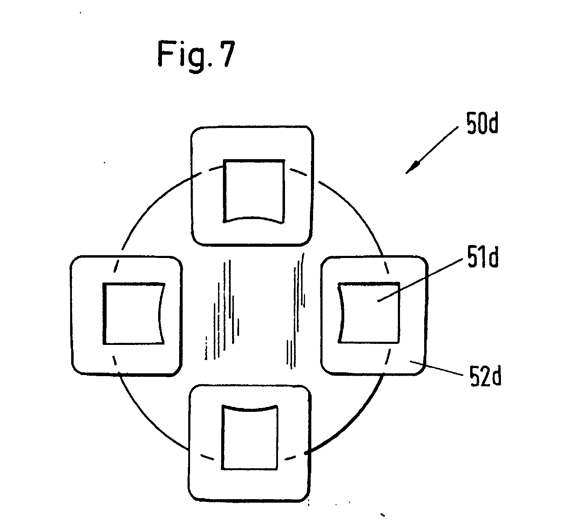

Es versteht sich von selbst, dass die anhand von Fig. 6, Fig. 7 und Fig. 8 erläuterte Einmalpumpe nicht zwingend als Zahradpumpe ausgebildet sein muss, sondern selbstverständlich auch als Zentrifugalpumpe oder als Pumpe eines anderen Typs ausgebildet sein kann.

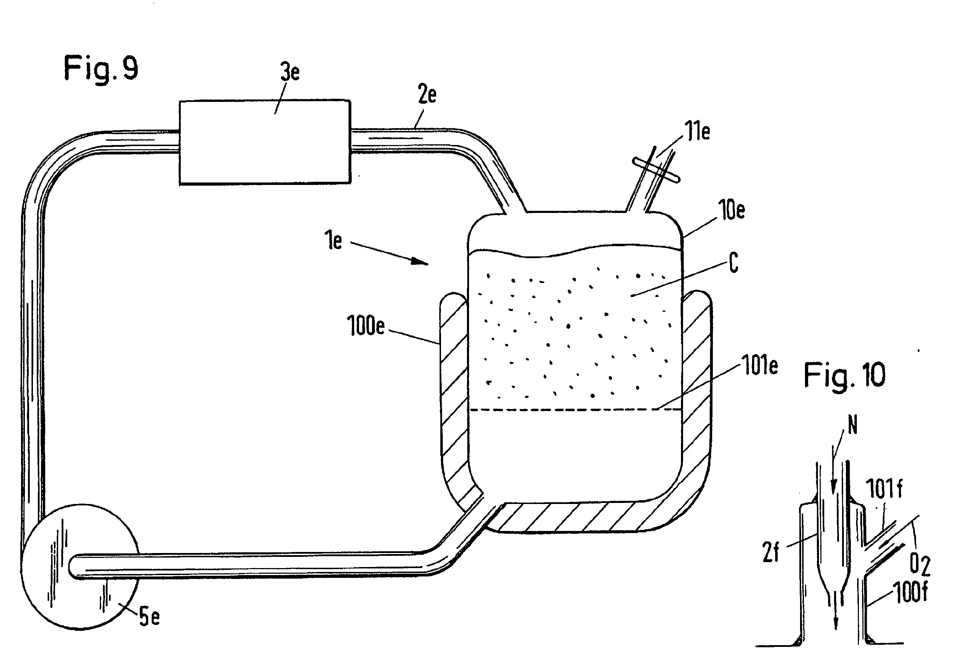

In Fig. 9 ist ein weiteres Ausführungsbeispiel eines erfindungsgemässen Bioreaktors dargestellt, bei welchem der Reaktionsbehälter 1e einen flexiblen Beutel, beispielweise einen Folienbeutel 10e umfasst, welcher in einer formstabilen Aufnahme aufgenommen wird, die beispielsweise als Wärmemantel 100e ausgebildet sein kann. Der Wärmemantel 100e verleiht dem Folienbeutel 10e die notwendige Stabilität und hält den Beutelinhalt auf einer gewünschten Temperatur. Ferner erkennt man einen Gasauslass 11e sowie eine Zufühleitung 2e für die Nährlösung N. Die Nährlösung N ist zuvor bereits mit Hilfe eines Oxygenators 3e mit Sauerstoff versetzt bzw. von Kohlendioxid befreit worden. Gegebenenfalls kann auch noch ein Reservoir (nicht dargestellt) mit Nährlösung N vorgesehen sein, aus welchem die Nährlösung mit Hilfe der Einmalpumpe 5e bzw. einer Pumpe 5e mit Einmalteilen gefördert wird.

In dem Folienbeutel 10e kann eine semipermeable Membran 101e vorgesehen sein, welche für die Nährlösung N durchlässig ist, jedoch nicht für die im Folienbeutel angeordneten Zellen C. Dadurch werden empfindliche Zellen nicht den relativ hohen Scherkräften in der Pumpe bzw. im Oxygenator ausgesetzt. Damit die semipermeable Membran 101e nicht verstopfen kann, kann ab und zu die Strömungsrichtung der Nährlösung umgekehrt werden. Andererseits könnte die Membran 101e auch senkrecht angeordnet sein. Der Raum, in welchem sich die Zellen C befinden kann auch mit "Microcarriers" gefüllt sein, um Zellen, die eine Verankerung benötigen (sogenannte "anchorage dependent cells") züchten zu können.

Fig. 10 zeigt schliesslich ein Ausführungsbeispiel, wie der Einlass des Reaktionsbehälter ausgebildet sein kann. Wenn der Einlass so ausgebildet ist wie in Fig. 10 gezeigt, so kann auf einen (aufwändigeren) Oxygenator verzichtet werden und der Nährlösung N nach dem Venturi-Prinzip ein gasförmiges Medium, beispielsweise Sauerstoff, beigemischt werden. In diesem Fall könnte auf eine Pumpe zur Zuführung des gasförmigen Mediums verzichtet werden. Natürlich kann die Gaszufuhr auch in bekannter Weise getrennt erfolgen, z.B. mittels einer hierfür vorgesehenen Membranpumpe. Die Lösung mit einer separaten Membranpumpe für die Gaszufuhr ist zwar aufwändiger, gestattet aber dafür auch eine bessere und vor allen Dingen eine von der Nährlösungszufuhr unabhängige Einstellung der Gaszufuhr.

In Fig. 10 erkennt man daher das Ende 2f der Zuführleitung, welches in einem Ansatzstück 100f in den Folienbeutel 10f einmündet. In das Ansatzstück mündet aber auch ein Kanal 101f, durch welchen bei der Zufuhr von Nährlösung N dann das gasförmige Medium, z.B. Sauerstoff, der Nährlösung beigemischt wird.

Schliesslich ist noch anzumerken, dass bei sämtlichen erläuterten Ausführungsbeispielen nicht nur die Pumpe oder Teile davon, sondern auch sämtliche anderen Bestandteile des Bioreaktors, die mit dem Medium in Kontakt kommen, als Einmalteile ausgebildet sein können. Insbesondere können die Reaktoren auch schon assembliert und steril verpackt geliefert werden, sodass keine Kontaminationsgefahr besteht, weil die der Kontaminationsgefahr ausgesetzten Teile alle nach einmaliger Anwendung ersetzt werden.

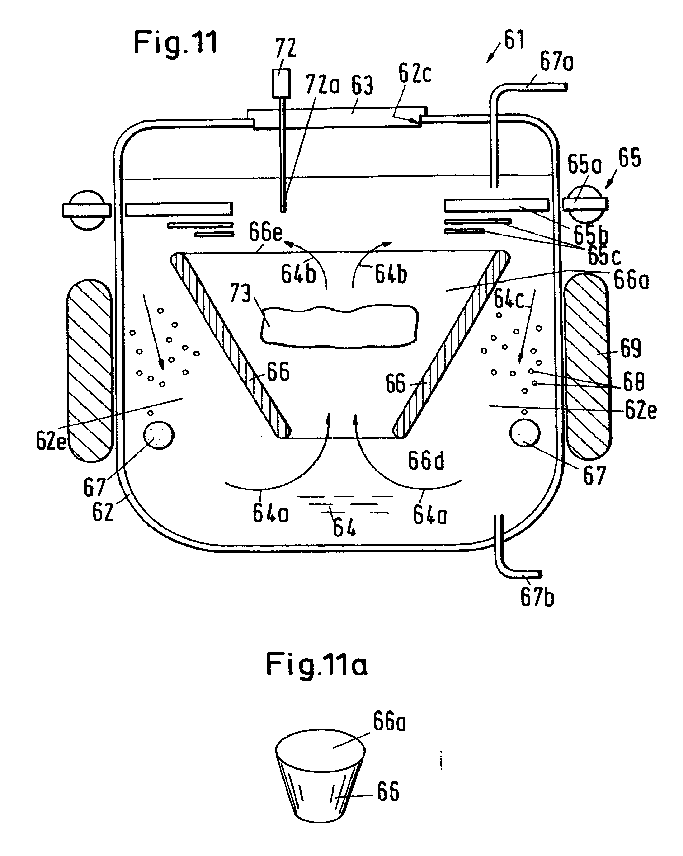

Der in Fig. 11 dargestellte Bioreaktor 61 umfasst einen Behälter 62, welcher oben eine mit einem Verschluss 63 verschliessbare Öffnung 62c aufweist. Im Innenraum des Behälter 62 ist ein kegelstumpfförmig, als Hohlkörper ausgebildetes Flussleitmittel 66 angeordnet, dessen Querschnittsfläche sich gegen oben vergrössert. Der Innenraum des Behälters 62 ist weitgehend mit einer Flüssigkeit 64 gefüllt, welche vom Flügelrad 65c des Motors 65 in eine Zirkulationsströmung versetzt wird, sodass die Flüssigkeit 64 die mit den Pfeilen 64a, 64b, 64c dargestellte Strömungsrichtung aufweist. Die in Richtung der Pfeile 64a strömende Flüssigkeit tritt von unten mit relativ hoher Flussgeschwindigkeit über die Eintrittsöffnung 66d in den Innenraum 66a des Flussleitmittels 66 ein, strömt im Innenraum 66a mit abnehmender Geschwindigkeit nach oben, und tritt oben durch die Austrittsöffnung 66e, wie mit den Pfeilen 64b dargestellt, mit relativ geringer Flussgeschwindigkeit wieder aus dem Innenraum 66a aus. Im Innenraum 66a reduziert sich die Fliessgeschwindigkeit auf Grund des sich gegen oben erweiternden Querschnittes. Der Innenraum 66a bildet den ersten Strömungsraum. Ist der Durchmesser der Austrittsfläche 66e beispielsweise doppelt so gross wie der Durchmesser der Eintrittsfläche 66d, so entspricht die Geschwindigkeit an der Austrittsfläche 66e einem Viertel der Geschwindigkeit an der Eintrittsfläche 66d. Die durch die Fliessgeschwindigkeit bewirkte Auftriebskraft beträgt an der Austrittsfläche 66e noch einen Sechzehntel derjenigen an der Eintrittsfläche 66d. Das im Innenraum 66a angeordnete Gut 73 wird durch die nach oben strömende Flüssigkeit in einer Gleichgewichtslage gehalten, wobei sich die Schwebehöhe beziehungsweise die Gleichgewichtslage von Auftriebskraft und Schwerkraft auf Grund des Gewichtes und der Angriffsfläche des Guts von selbst einstellt.

Oberhalb des Flussleitmittels 66 ist eine Pumpe 65 angeordnet, welche einen ausserhalb des Behälters 62 angeordneten Eisenstator 65a und einen innerhalb des Behälters 62 angeordneten Rotor 65b umfasst. Ein Flügelrad 65c ist mit dem Rotor 65b fest verbunden. Eine derartige Vorrichtung umfassend einen Stator sowie einen mit magnetisch wirkenden Kräften gehaltenen und angetriebenen Rotor wird auch als lagerloser Motor bezeichnet, und ist einem Fachmann bekannt, beispielsweise aus der Druckschrift WO 96/31934.

Das Flügelrad 65c erzeugt die mit dem Pfeilen 64a, 64b, 64c dargestellte Kreisströmung. Zwischen dem Behälter 62 und dem Flussleitmittel 66 ist ein Innenraum 62e ausgebildet, auch als zweiter Strömungsraum bezeichnet, mit einer sich von oben nach unten erweiternden Querschnittsfläche. Die hat zur Folge, dass die in Strömungsrichtung 64c von oben nach unten strömende Flüssigkeit eine gegen unten abnehmende Fliessgeschwindigkeit aufweist.

Im Innenraums 62e ist unten ein ringförmiger Verteiler 67 angeordnet, durch welchen Luft oder Sauerstoff zur Begasung der Flüssigkeit 64 eingeleitet wird, welche innerhalb der Flüssigkeit 64 Luftblasen 68 bilden, welche aufzusteigen bestrebt sind. Durch die in Richtung 64c von oben nach unten strömende Flüssigkeit wird das Aufsteigen der Luftblasen 68 verzögert oder verhindert, was den Gasaustausch zur Flüssigkeit 64 fördert.

Der Behälter 62 ist aussen von einer ringförmigen Heizvorrichtung 69 umgeben. Der Innenraum des Behälters 62 wird über eine Zuleitung 67a und eine Ableitung 67b mit einer Nährflüssigkeit versorgt. Eine Messsonde 72 mit Sondenkopf 72a ermöglich beispielsweise eine Überwachung des pH-Wertes oder der Temperatur der Flüssigkeit 64.

Der in Figur 11 dargestellte Bioreaktor 61 weist den Vorteil auf, dass das Gut 73 über den einen grossen Durchmesser aufweisenden Verschluss 63 leicht zugänglich ist.

Fig. 11a zeigt eine perspektivische Darstellung des Flussleitmittels 66 mit Innenraum 66a.

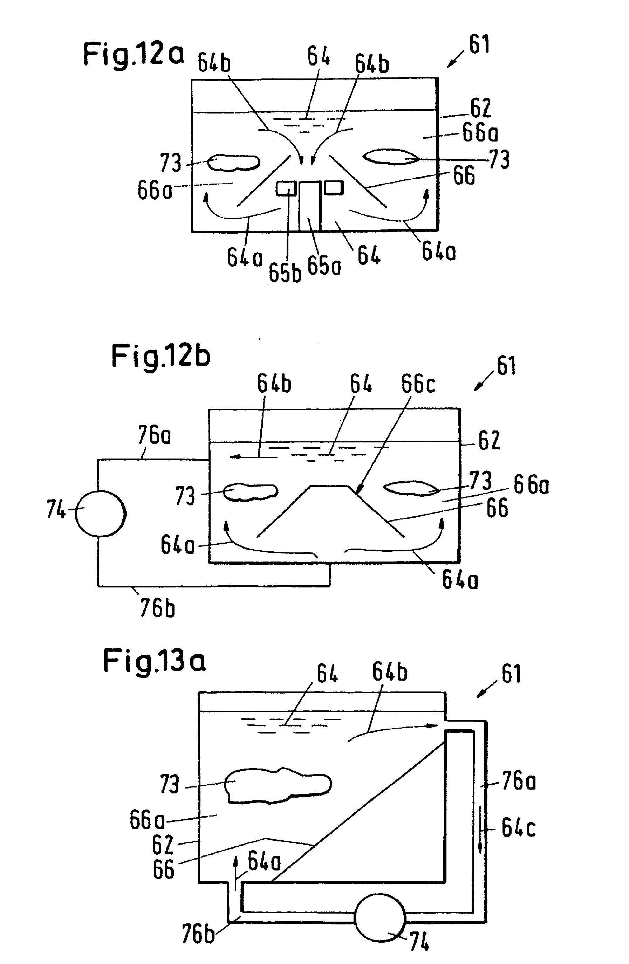

Fig. 12a zeigt schematisch einen Längsschnitt durch ein weiteres Ausführungsbeispiel eines Bioreaktors 61, welcher sich bezüglich dem in Fig. 11 dargestellten Beispiel dadurch unterscheidet, dass das Flussleitmittel 66 umgekehrt, das heisst mit einem sich gegen unten erweiternden Querschnitt angeordnet ist. Die Pumpe 65 umfassend den Eisenstator 65a und den rotierbaren Teil 65b mit Flügelrad 65c, bewirkt in der Flüssigkeit 64 eine Strömung in Richtung 64a, 64b. Der Innenraum 66a, in welchem die Flüssigkeit nach oben strömt, und in welchem das Gut 73 gehalten ist, befindet sich zwischen dem Flussleitmittel 66 und der Aussenwand des Behälters 62.

Fig. 12b zeigt schematisch einen Längsschnitt durch ein weiteres Ausführungsbeispiel eines Bioreaktors 61, welcher sich bezüglich dem in Fig. 12a dargestellten Beispiel dadurch unterscheidet, dass das Flussleitmittel 66 oben dicht ausgestaltet ist, und dass die Fluidpumpe 74 ausserhalb des Behälters 62 angeordnet ist, wobei die Pumpe 74 über Leitungen 76a, 76b fluidleitend mit dem Innenraum des Behälters 62 verbunden ist. Das in Richtung 64a strömende Fluid tritt von unten in den Innenraum 66a ein und umströmt das Gut 73.

Fig. 13a zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Das Flussleitmittel 66 ist nur auf der einen Innenseite des Behälters 62 sich gegen oben erweiternd ausgestaltet. Das Gut 73 wird durch die in Richtung 64a, 64b, 64c zirkulierende Flüssigkeit im Innenraum 66a in der Schwebe gehalten.

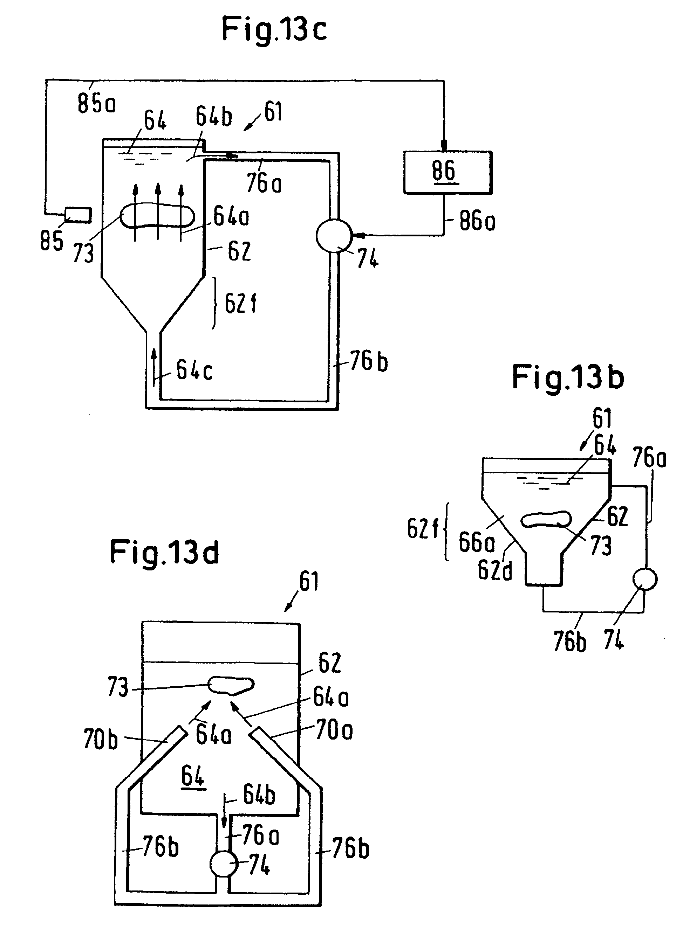

Fig. 13b zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Der Behälter 62 weist entlang eines Abschnitts 62f eine sich gegen oben erweiternde Behälterwand 62d auf. Entlang dieses Abschnittes 62f bildet sich eine Strömung mit einer sich gegen oben verringernden Fliessgeschwindigkeit auf, sodass der Innenraum 66a zum schwebenden Halten des Guts 73 entlang dieses Abschnittes 62f ausgebildet ist.

Fig. 13c zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Die Leitung 76a münden in einem sich gegen oben erweiternden Abschnitt 62f in den Behälter 62 ein. Nachfolgend ist ein zylinderförmig ausgestalteter Behälterabschnitt 62 angeordnet, innerhalb welchem sich eine lineare Strömung 64a ausbildet und innerhalb welchem das Gut 73 angeordnet ist. Mit einem Sensor 85 wird die Höhenlage des Gut 73 überwacht. Eine Regelvorrichtung 86 ist über eine elektrische Leitung 85a, 86a signalleitend mit dem Sensor 85 sowie der Pumpe 74 verbunden. Die Drehzahl der Pumpe 74 wird derart geregelt, dass das Gut 73 in Bereich des Sensors 85 verbleibt.

Fig. 13d zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Mehrere, beispielsweise drei Düsen 70a, 70b münden innerhalb des Behälters 62 auf das Gut 73 ausgerichtet, wobei die mit 64a dargestellte Strömungsrichtung eine gegen oben reduzierte Fliessgeschwindigkeit aufweist, sodass das Gut 73 von dieser Strömung getragen wird und selbstständig eine Gleichgewichtslage findet.

In allen in den Figuren 11 bis 13d dargestellten Bioreaktoren 61 wird das Gut 73 mit demselben Verfahren in einem schwebenden Zustand gehalten, nämlich dadurch, dass das Gut 73 mit einem Fluid beaufschlag wird, dessen Strömung entgegen der auf das Gut 73 wirkenden Schwerkraft wirkt, derart, dass das Gut 73 in der Schwebe gehalten wird. In den Ausführungsbeispielen gemäss den Figuren 11, 12a, 12b, 13a, 13b und 13d weist das Fluid im Innenraum 66a mit zunehmender Höhe eine geringere Strömungsgeschwindigkeit auf. Im Ausführungsbeispiel gemäss Fig. 13c wird die Geschwindigkeit des Fluides mit einem Sensor 85 in Abhängigkeit der Lage des Guts 73 geregelt.

Im Ausführungsbeispiel gemäss Fig. 11 wird innerhalb des Behälters 62 eine nach unten fliessende Strömung 64c erzeugt, wobei in diese Strömung 64c ein gasförmiges Fluid wie Luft oder Sauerstoff eingeleitet wird. Die Fliessgeschwindigkeit der Strömung 64c kann derart gewählt werden, dass das eingeleitete, gasförmige Fluid im Behälter 62 verlangsamt oder gar nicht mehr aufsteigt.

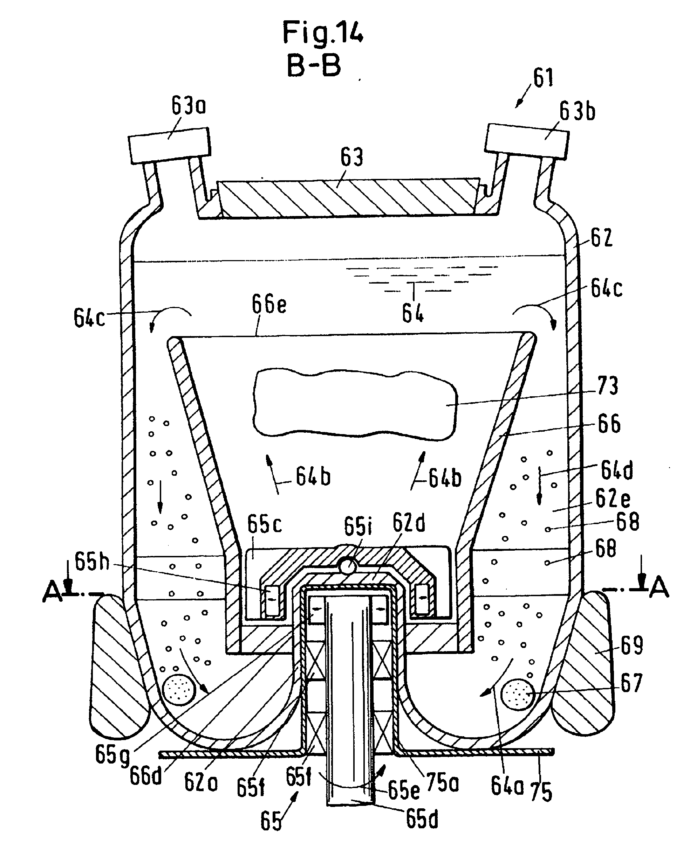

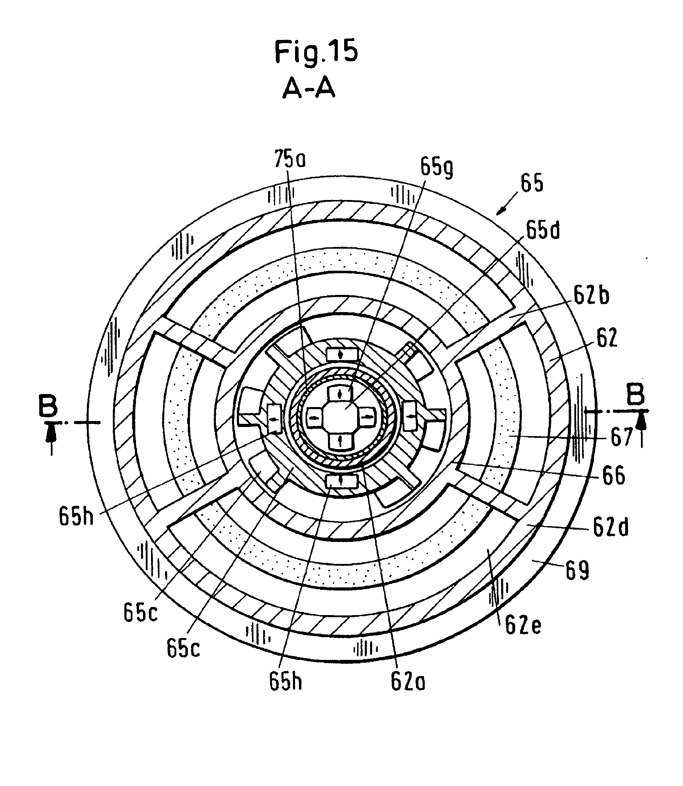

Fig. 14 zeigt mit einem Längsschnitt entlang der Linie B-B gemäss Fig. 15 ein weiteres Ausführungsbeispiel eines Bioreaktors 61. Ansonst ähnlich ausgestaltet wie der in Fig. 11 dargestellte Bioreaktor 61, ist im Bioreaktor 61 gemäss Fig. 14 die Pumpe 65 unten, im Bereich der Eintrittsöffnung 66d des Flussleitmittels 66 angeordnet. Innerhalb des Behälters 62 ist ein Flügelrad 65c drehbar auf einem Spurlager 65i angeordnet, wobei das Spurlager 65i auf der Behälterwand 62d aufliegt. Innerhalb des aus einem Kunststoff bestehenden Flügelrad 65c ist eine Mehrzahl über den Umfang verteilt angeordneter Permanentmagnete 65h eingegossen. Ausserhalb des Behälters 62 ist eine in Richtung 65e drehbar gelagerte Magnetkupplung angeordnet, welche zwei Lager 65f und einen ringförmigen Permanentmagneten 65g umfasst. Die rotierbare Welle 65d ist von einem nicht dargestellten Motor angetrieben. Eine Standvorrichtung 75 bildet einen Spalttopf 75a, welcher zylinderförmig ausgestaltet ist und zwischen den beiden Permanentmagneten 65g, 65h verlaufend angeordnet ist. Die Behälterwand 62d bildet beim Spalttopf 75a einen Spalttopfabschnitt 62a aus. Die magnetische Kupplung umfassend die Permanentmagnete 65h, 65i bewirkt, dass die Drehbewegung der rotierbaren Welle 65d auf das Flügelrad 65c übertragen wird, und dass das Flügelrad 65c bezüglich einem Kippen gehalten ist. Somit ist das Flügelrad 65c passiv magnetisch gehalten.

Der Behälter 62 sowie das darin drehbar gelagerte Flügelrad 65c ist vorzugsweise zur einmaligen Verwendung als Einmalprodukt ausgestaltet. Der Behälter 62 kann auf die Heizvorrichtung 69 sowie auf den Spalttopf 75a aufgesetzt werden, sodass der Behälter 62 sicher gehalten ist und das Flügelrad 65c über die drehbar gelagerte Magnetkupplung antreibbar ist.

Der Behälter 62 kann, wie in Fig. 14 dargestellt, zusätzliche Öffnungen 63a, 63b aufweisen, beispielsweise für Messsonden.

Fig. 15 zeigt einen Querschnitt entlang der Linie A-A gemäss Fig. 14. Im Zentrum ist die rotierbare Welle 65d angeordnet, an welcher vier beabstandet angeordnete Permanentmagnete 65g befestigt sind. Der Behälter 62 bildet mit dessen Behälterwand 62d einen Spalttopfabschnitt 62a aus. Zwischen dem Spalttopfabschnitt 62a und der rotierbaren Welle 65d mit Permanentmagnet 65g ist der Spalttopf 75a angeordnet. Der Spalttopfabschnitt 62a ist vom Flügelrad 65c umgeben, innerhalb welchem vier Permanentmagnete 65h angeordnet sind, wobei deren Polarisierung, mit Pfeilen dargestellt, derjenigen der Permanentmagnete 65g angepasst ausgerichtet ist. Das Flussleitmittel 66 ist über Fluidleitteile 62d mit der Aussenwand des Behälters 62 verbunden. Zwischen dem Flussleitmittel 66 und der Aussenwand des Behälters 62 ist der sich von oben nach unten erweiternde Strömungsraum 62e angeordnet. Zudem ist der ringförmig ausgestaltete Verteiler 67 dargestellt.

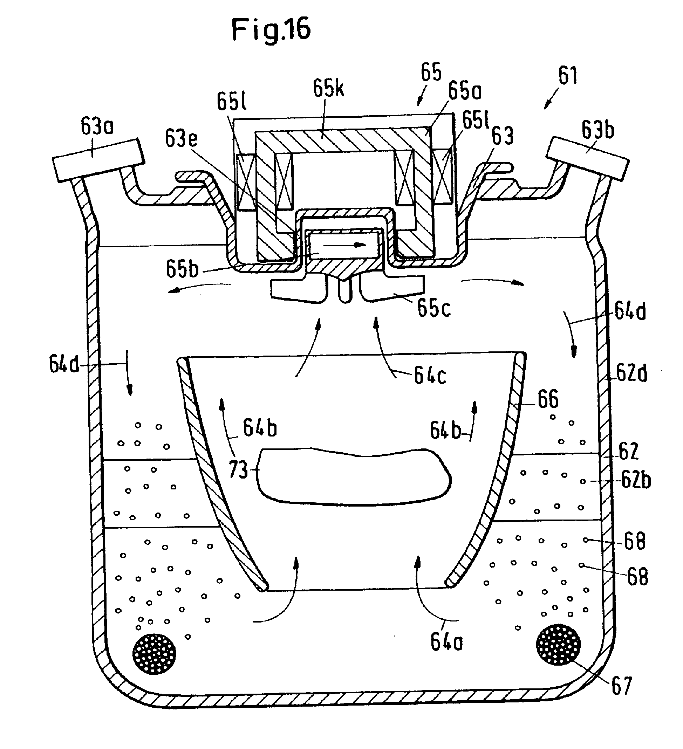

Fig. 16 zeigt einen Längsschnitt durch ein weiteres Ausführungsbeispiel eines Bioreaktors 61. Im Unterschied zu dem in Fig. 11 dargestellten Bioreaktor 61, ist im Bioreaktor 61 gemäss Fig. 16 die Pumpe 65 im Verschluss 63 angeordnet und als Zentrifugalpumpe ausgestaltet. Die Pumpe 65 ist als Spaltrohrmotor ausgestaltet und umfasst den fest angeordneten Eisenstator 65a sowie den berührungslos drehbar gelagerten, rotierbaren Teil 65b, ausgebildet als Permanentmagnet, welcher fest mit dem Flügelrad 65c verbunden ist. Der Eisenstator 65a umfasst ein Weicheisen 65k, welches von einer Mehrzahl von Spulen 65I umgeben ist. Die Spulen 65I sind derart angeordnet und ansteuerbar, dass der rotierbare Teil 65b berührungslos antreibbar und gehalten ist. Der Verschluss 63 weist einen Spalttopfabschnitt 63e auf, welcher im Spalt zwischen dem Eisenstator 65a und dem Permanentmagnet 65b angeordnet ist.

Eine derartige Vorrichtung umfassend einen Stator sowie einen mit magnetisch wirkenden Kräften gehaltenen und angetriebenen Rotor wird auch als Tempelmotor bezeichnet, und ist einem Fachmann bekannt, beispielsweise aus der Druckschrift WO 96/31934, insbesondere aus deren Figur 12.

Das Flussleitmittel 66 ist über Fluidleitteile 62b fest mit der Behälterwand 62d verbunden. Das Flussleitmittel 66 weist einen sich gegen oben bauchförmig erweiternden Querschnitt auf. Das Flussleitmittel 66 ist in einer Vielzahl weiterer Ausführungsformen ausgestaltbar, derart, dass sich eine gegen oben vergrössernde Querschnittsfläche ergibt.

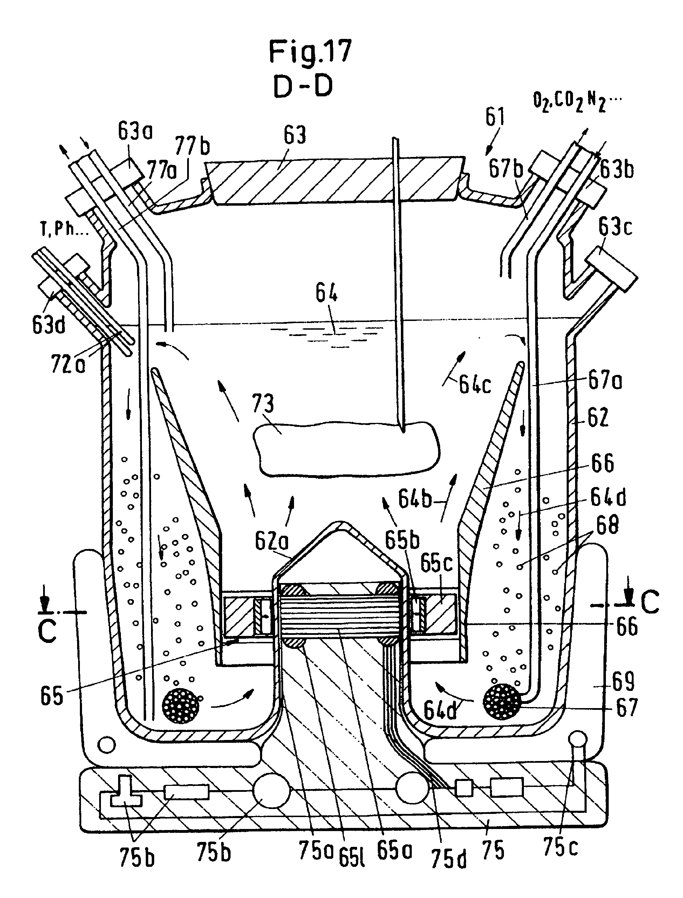

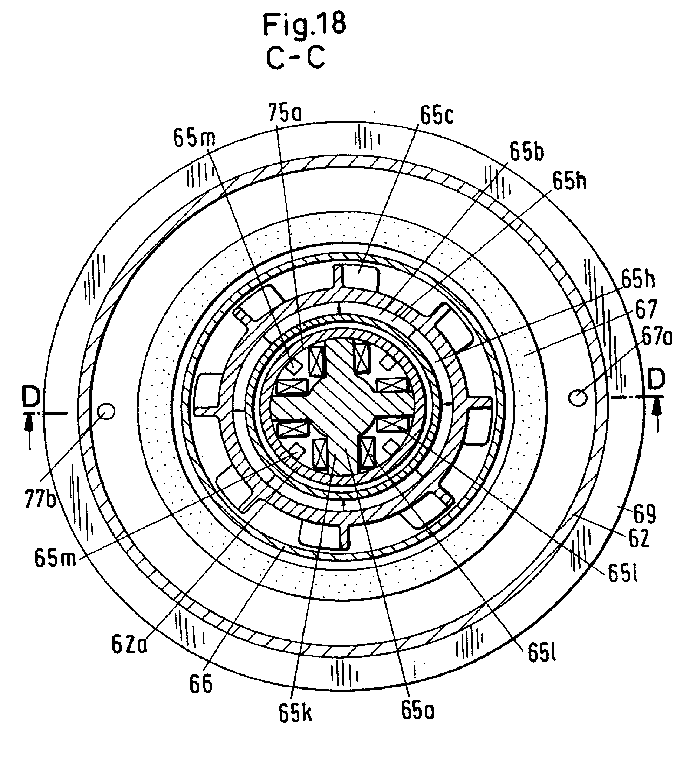

Fig. 17 zeigt mit einem Längsschnitt entlang der Linie D-D gemäss Fig. 18 ein weiteres Ausführungsbeispiel eines Bioreaktors 61. Im Unterschied zu dem in Fig. 14 dargestellten Bioreaktor 61 weist die Pumpe 65 ein vollständig magnetisch gelagertes und angetriebenes rotierbares Teil 65b mit Flügelrad 65c auf. Aus dem in Fig. 18 dargestellten Querschnitt entlang der Schnittlinie C-C ist der lagerlose Antrieb der Pumpe 65 im Detail dargestellt. Die Funktionsweise eines derartigen Antriebs ist beispielsweise in der Druckschrift WO 98/59406 offenbart. Der Eisenstator 65a ist als kreuzförmiges Blechpaket 65k ausgestaltet, an dessen Arme Spulen 65l angeordnet sind. Durch eine entsprechende Ansteuerung der Spulen 65l ist damit ein sich drehendes, magnetisches Feld erzeugbar. Das rotierbare Teil 65b umfasst vier in Umfangsrichtung angeordnete Permanentmagnete 65h, wobei zwei benachbarte Permanentmagnete 65h jeweils in entgegengesetzter Richtung polarisiert sind. Diese Permanentmagnete 65h sind im Flügelrad 65c bzw. in den Pumpenschaufeln 65c eingegossen bzw. gekapselt. Im Stator sind Sensoren 65m angeordnet, welche die Lage der Permanentmagnete 65h messen. In der Standvorrichtung 75 sind elektronische Komponenten 75b angeordnet, mit einer elektrischen Zuleitung 75d für die Spulen 65l des Motors sowie mit einer elektrischen Zuleitung 75c für die Heizung 69. Zudem sind elektrische Leitungen angeordnet, welche die Sensoren 65m mit den elektronischen Komponenten 75b verbindet. Die Spulen 65l werden derart angesteuert, dass das rotierbare Teil 65 mit Pumpenschaufeln 65c berührungslos gehalten und angetrieben ist. Die Pumpe 65 bildet eine Axialpumpe. Zwischen dem Eisenstator 65a und dem rotierbaren Teil 65b ist der Spalttopf 75a und der Spalttopfabschnitt 62a der Behälterwand 62d angeordnet.

Die Standvorrichtung 75 sowie die Heizung 69 bilden eine feste Unterlage und Halterung, in welche der Behälter 62 einführbar ist. Diese Anordnung weist den Vorteil auf, dass der Behälter 62 sehr einfach auf die Standvorrichtung 75 mit Heizung 69 gestellt werden kann, und die Axialpumpe 65 danach sofort betreibbar ist, ohne zusätzliche Handgriffe zu tätigen. Der Behälter 62 mit rotierbarem Teil 65b und Pumpenschaufeln 65c ist als Einmalprodukt ausgestaltet, wogegen die teuren Komponenten der Standvorrichtung 75 und der Heizung 69 belieb oft verwendbar sind. Zudem muss die Standvorrichtung 75 sowie die Heizung 69 nicht steril sein, sodass kein aufwendiger Reinigungsprozess erforderlich ist. Vorteile dieser Anordnung sind die Tatsachen, dass der Innenraum des Behälters 62 problemlos steril gehalten werden kann, dass der Behälter 62 kostengünstig herstellbar ist, und dass die Standvorrichtung 75 ohne aufwendigen Reinigungsprozess und somit kostengünstig betreibbar ist.

Im Behälter 62 gemäss Fig. 17 verlaufen durch den Verschluss 63b die Zuund Ableitung 67a, 67b für Gase wie O₂, CO₂, N₂, wobei die Zuleitung 67a Fluid leitend mit dem ringförmigen Verteiler 67 verbunden ist. Durch den Verschluss 63a verlaufen die Zu- und Ableitung 77a, 77b für das Nährmedium. Zudem verlaufen durch den Verschluss 63d Sonden mit Sondenköpfen 72a, beispielsweise zur Messung von Temperatur oder pH-Wert.

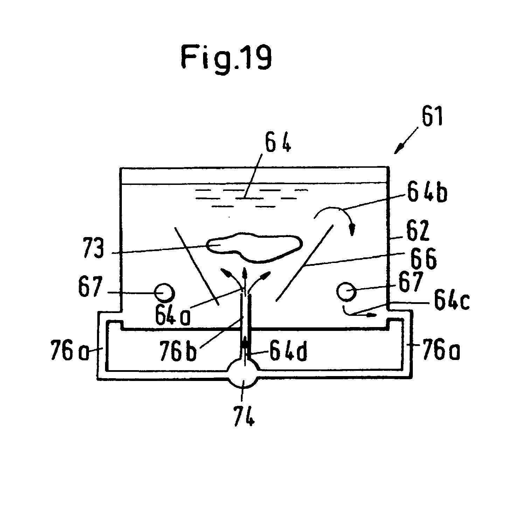

Fig. 19 zeigt schematisch einen Längsschnitt durch ein weiteres Ausführungsbeispiel eines Bioreaktors 61, welcher ebenfalls eine ausserhalb des Behälters 62 angeordnete Fluidpumpe 74 aufweist, welche über Leitungen 76a, 76b fluidleitend mit dem Innenraum verbunden ist. Die Leitung 76b münden in den sich gegen oben erweiternden Abschnitt des Flussleitmittels 66 ein. Über die im Bodenbereich des Behälters 62 angeordneten Leitungen 76a wird das Fluid zur Fluidpumpe 74 geleitet, sodass das Fluid das mit Pfeilen 64a, 64b, 64c angedeutete Strömungsverhalten aufweist.

## Patentansprüche

1. Verfahren zum schwebenden Halten eines Gutes (73) in einem Bioreaktor (61), wobei unter dem Begriff "Gut" sowohl ein Gewebeträger an sich, als auch ein Gewebeträger mit angelagerten Zellen, oder falls der Gewebeträger abbaubar ausgestaltet ist, eine künstlich erzeugte Zellkultur bzw. ein künstlich erzeugtes Gewebeteil verstanden wird, **dadurch gekennzeichnet, dass** das Gut (73) mit einem Fluid beaufschlag wird, und dass die Strömung des Fluides derart der Schwerkraft oder dem Auftrieb entgegengesetzt wirkt, dass das Gut (73) in der Schwebe gehalten wird.

2. Verfahren nach Anspruch 1, **dadurch gekennzeichnet, dass** das Fluid in zur Schwerkraft oder zur Auftriebskraft entgegengesetzten Richtung eine zunehmend geringere Strömungsgeschwindigkeit aufweist.

3. Verfahren nach Anspruch 1 oder 2, **dadurch gekennzeichnet, dass** die Lage des Gutes(73) im Bioreaktor (61) mit einem Sensor (85) gemessen wird, und dass die Geschwindigkeit des Fluides im Bioreaktor (61) derart in Abhängigkeit der Lage des Gutes (73) geregelt wird, dass das Gut (73) in einer vorbestimmten Lage in der Schwebe gehalten wird.

4. Verfahren nach einem der vorhergehenden Ansprüche, **dadurch gekennzeichnet, dass** im Bioreaktor (61) zudem eine in Richtung der Schwerkraft fliessende Abwärtsströmung des Fluides erzeugt wird, und dass in dieses abwärts strömende Fluid ein gasförmiges Fluid, insbesondere Luft oder Sauerstoff, eingeleitet wird.

5. Verfahren nach Anspruch 4, **dadurch gekennzeichnet, dass** die Fliessgeschwindigkeit des abwärts strömenden Fluides derart gewählt wird, dass das eingeleitete, gasförmige Fluid verlangsamt oder gar nicht aufsteigt.

6. Bioreaktor (61) mit einem Behälter (62) für ein mit einem Fluid zu beaufschlagendes Gut (73), wobei unter dem Begriff "Gut" sowohl ein Gewebeträger an sich, als auch ein Gewebeträger mit angelagerten Zellen, oder falls der Gewebeträger abbaubar ausgestaltet ist, eine künstlich erzeugte Zellkultur bzw. ein künstlich erzeugtes Gewebeteil verstanden wird, umfassend einen ersten Strömungsraum (66a) welchem ein strömendes Fluid zuführbar ist, wobei der erste Strömungsraum (66a) derart ausgestaltet ist, dass das darin von unten nach oben strömende Fluid mit zunehmender Höhe eine kleinere Geschwindigkeit aufweist.

7. Bioreaktor (61) nach Anspruch 6 mit einem Behälter (62) für ein mit einem Fluid zu beaufschlagendes Gut (73), umfassend einen ersten Strömungsraum (66a) welchem ein strömendes Fluid zuführbar ist, sowie umfassend einen Sensor (85), welcher derart angeordnet und ausgestaltet ist, dass die Lage des beaufschlagten Gutes (73) im ersten Strömungsraum (66a) messbar ist, sowie umfassend eine mit dem ersten Strömungsraum (66a) fluidleitend verbundene Fluidfördervorrichtung (74), und umfassend eine Regelvorrichtung (86) welche mit dem Sensor (85) sowie der Fluidfördervorrichtung (74) Signal leitend verbunden ist.

8. Bioreaktor (61) nach Anspruch 6 oder 7, **dadurch gekennzeichnet, dass** der erste Strömungsraum (66a) sich von unten nach oben erweiternd ausgestaltet ist.

9. Bioreaktor (61) nach Anspruch 8, **dadurch gekennzeichnet, dass** der Behälter (62) einen Abschnitt (62f) mit einer sich gegen oben erweiternden Behälterwand (62d) aufweist, und dass dieser Abschnitt (62f) den ersten Strömungsraum (66a) ausbildet.

10. Bioreaktor (61) nach einem der Ansprüche 6 bis 9, **dadurch gekennzeichnet, dass** zumindest eine Fluidleitung (76b) in den ersten Strömungsraum (66a) mündet, vorzugsweise von unten oder bezüglich dem Strömungsraum (66a) seitlich angeordnet.

11. Bioreaktor (61) nach einem der Ansprüche 6 bis 10, **dadurch gekennzeichnet, dass** im Behälter (62) ein Flussleitmittel (66) angeordnet ist, welches den ersten Strömungsraum (66a) ausbildet, wobei das Flussleitmittel (66) derart ausgestaltet ist, dass sich der erste Strömungsraum (66a) von unten nach oben erweitert.

12. Bioreaktor (61) nach Anspruch 11, **dadurch gekennzeichnet, dass** das Flussleitmittel (66) als Hohlkörper (66b) ausgestaltet ist.

13. Bioreaktor (61) nach Anspruch 12, **dadurch gekennzeichnet, dass** der Hohlkörper (66b) einen sich gegen oben erweiternden Innenraum aufweist, welcher den ersten Strömungsraum (66a) ausbildet.

14. Bioreaktor (61) nach Anspruch 12, **dadurch gekennzeichnet, dass** der Hohlkörper (66b) eine sich gegen oben reduzierende Aussenkontur (66c) aufweist, und dass der Hohlkörper (66b) derart im Behälter (62) angeordnet ist, dass der sich von unten nach oben erweiternde erste Strömungsraum (66a) zwischen der Aussenkontur (66c) und der Behälterwand (62d) ausgebildet ist.

15. Bioreaktor (61) nach einem der Ansprüche 6 bis 14, **dadurch gekennzeichnet, dass** der Behälter (62) oben zumindest eine verschliessbare Öffnung (63, 63a, 63b, 63c) aufweist.

## Claims

1. A method for suspending a material (73) in a bioreactor (61), wherein the term "material" is understood to mean both a tissue carrier per se and a tissue carrier having accreted cells or, if the tissue carrier is designed as degradable, an artificially produced cell culture or an artificially produced tissue part, **characterised in that** the material (73) is acted on by a fluid; and **in that** the flow of the fluid acts opposite to gravity or to lift such that the material (73) is held in suspension.

2. A method in accordance with claim 1, **characterised in that** the fluid has an increasingly lower flow speed in the direction opposite to gravity or to the lifting force.

3. A method in accordance with claim 1 or claim 2, **characterised in that** the position of the material (73) in the bioreactor (61) is measured by a sensor (85); and **in that** the speed of the fluid in the bioreactor (61) is regulated in dependence on the position of the material (73) such that the material (73) is held in suspension in a predefined position.

4. A method in accordance with any one of the preceding claims, **characterised in that** a downward flow of the fluid, flowing in the direction of gravity, is additionally produced in the bioreactor (61); and **in that** a gaseous fluid, in particular air or oxygen, is introduced into this downwardly flowing fluid.

5. A method in accordance with claim 4, **characterised in that** the flow speed of the downwardly flowing fluid is selected such that the introduced gaseous fluid decelerates or does not rise at all.

6. A bioreactor (61) having a container (62) for a material (73) to be acted on by a fluid, wherein the term "material" is understood to mean both a tissue carrier per se and a tissue carrier having accreted cells or, if the tissue carrier is designed as degradable, an artificially produced cell culture or an artificially produced tissue part, including a first flow space (66a) to which a flowing fluid can be supplied, wherein the first flow space (66a) is designed such that the fluid flowing from bottom to top therein has a lower speed as the height increases.

7. A bioreactor (61) in accordance with claim 6, having a container (62) for a material (73) to be acted on by a fluid, including a first flow space (66a) to which a flowing fluid can be supplied, as well as including a sensor (85) which is arranged and designed such that the position of the material (73) acted on in the first flow space (66a) can be measured as well as including a fluid conveying apparatus (74) connected to the first flow space (66a) in a fluid-conductive manner, and including a regulation apparatus (86) which is connected to the sensor (85) and to the fluid conveying apparatus (74) in a signal conductive manner.

8. A bioreactor (61) in accordance with claim 6 or claim 7, **characterized in that** the first flow space (66a) is designed in a manner expanding from bottom to top.

9. A bioreactor (61) in accordance with claim 8, **characterised in that** the container (62) has a section (62f) having an upwardly expanding container wall (62d); and **in that** this section (62f) forms the first flow space (66a).

10. A bioreactor (61) in accordance with any one of the claims 6 to 9, **characterised in that** at least one fluid line (76b) opens into the first flow space (66a), preferably from below or arranged laterally with respect to the flow space (66a).

11. A bioreactor (61) in accordance with any one of the claims 6 to 10, **characterised in that** a flow conductive means (66) is arranged in the container (62) and forms the first flow space (66a), with the flow conductive means (66) being designed such that the first flow space (66a) expands from bottom to top.

12. A bioreactor (61) in accordance with claim 11, **characterised in that** the flow conductive means (66) is designed as a hollow body (66b).

13. A bioreactor (61) in accordance with claim 12, **characterised in that** the hollow body (66b) has an upwardly expanding inner space which forms the first flow space (66a).

14. A bioreactor (61) in accordance with claim 12, **characterised in that** the hollow body (66b) has an upwardly reducing outer contour (66c); and **in that** the hollow body (66b) is arranged in the container (62) such that the first flow space (66a) expanding from bottom to top is formed between the outer contour (66c) and the container wall (62d).

15. A bioreactor (61) in accordance with any one of the claims 6 to 14, **characterised in that** the container (62) has at least one closable opening (63, 63a, 63b, 63c) at the top.

## Revendications

1. Procédé pour le maintien en suspension d'un produit (73) dans un bioréacteur (61), où on comprend par le terme "produit" à la fois un support de tissu en tant que tel, et également un support de tissu avec des cellules en adhésion ou bien, dans le cas où le support de tissu est réalisé d'une manière dégradable, une culture de cellules produite artificiellement respectivement une partie de tissu produite artificiellement, **caractérisé en ce que** le produit (73) est chargé par un fluide, et **en ce que** l'écoulement du fluide s'oppose de telle sorte à la gravité ou à la force ascensionnelle que le produit (73) est maintenu en suspension.

2. Procédé selon la revendication 1, **caractérisé en ce que** le fluide présente dans la direction opposée à la gravité ou à la force ascensionnelle une vitesse d'écoulement de plus en plus réduite.

3. Procédé selon la revendication 1 ou 2, **caractérisé en ce que** la position du produit (73) dans le bioréacteur (61) est mesurée avec un capteur (85), et **en ce que** la vitesse du fluide dans le bioréacteur (61) est réglée de telle sorte en fonction de la position du produit (73) que le produit (73) est maintenu en suspension dans une position prédéterminée.

4. Procédé selon l'une des revendications précédentes, **caractérisé en ce qu'**est produit dans le bioréacteur (61) de plus un écoulement descendant du fluide s'écoulant dans la direction de la gravité, et **en ce qu'**est introduit dans ce fluide s'écoulant vers le bas un fluide gazeux, en particulier de l'air ou de l'oxygène.

5. Procédé selon la revendication 4, **caractérisé en ce que** la vitesse d'écoulement du fluide s'écoulant vers le bas est sélectionnée de telle sorte que le fluide gazeux introduit est ralenti ou ne monte pas.

6. Bioréacteur (61) avec un contenant (62) pour un produit (73) à charger par un fluide, où on comprend par le terme "produit" à la fois un support de tissu en soi, et également un support de tissu avec des cellules en adhésion, ou dans le cas où le support de tissu est réalisé d'une manière dégradable, une culture de cellules produites artificiellement ou une partie de tissu produit artificiellement, comprenant un premier espace d'écoulement (66a) auquel un fluide coulant peut être amené, où le premier espace d'écoulement (66a) est réalisé de telle sorte que le fluide s'écoulant dans celui-ci de bas en haut, au fur et à mesure qu'augmente la hauteur, présente une vitesse plus petite.

7. Bioréacteur (61) selon la revendication 6, avec un contenant (62) pour un produit (73) à charger par un fluide, comprenant un premier espace d'écoulement (66a) auquel un fluide coulant peut être amené, et comprenant un capteur (85) qui est disposé et configuré de façon que la position du produit chargé (73) dans le premier espace d'écoulement (66a) puisse être mesurée, et comprenant un dispositif de convoyage de fluide (74) relié fluidement au premier espace d'écoulement (66a) et comprenant un dispositif de régulation (86) qui est relié par signaux au capteur (85) et au dispositif de convoyage de fluide (74).

8. Bioréacteur (61) selon la revendication 6 ou 7, **caractérisé en ce que** le premier espace d'écoulement (66a) est réalisé en s'élargissant de bas en haut.

9. Bioréacteur (61) selon la revendication 8, **caractérisé en ce que** le contenant (62) présente une section (62f) avec une paroi de contenant (62d) s'élargissant vers le haut, et **en ce que** cette section (62f) forme le premier espace d'écoulement (66a).

10. Bioréacteur (61) selon l'une des revendications 6 à 9, **caractérisé en ce qu'**au moins un conduit de fluide (76b) débouche dans le premier espace d'écoulement (66a), et est disposé de préférence depuis le bas ou latéralement relativement à l'espace d_{'}écoulement (66a).

11. Bioréacteur (61) selon l'une des revendications 6 à 10, **caractérisé en ce qu'**un moyen de guidage d'écoulement (66) est disposé dans le contenant (62), qui forme le premier espace d'écoulement (66a), où le moyen de guidage d'écoulement (66) est réalisé de telle sorte que le premier espace d'écoulement (66a) s'élargit de bas en haut.

12. Bioréacteur (61) selon la revendication 11, **caractérisé en ce que** le moyen de guidage d'écoulement (66) est réalisé comme corps creux (66b).

13. Bioréacteur (61) selon la revendication 12, **caractérisé en ce que** le corps creux (66b) présente un espace intérieur s'élargissant vers le haut qui forme le premier espace d'écoulement (66a).

14. Bioréacteur (61) selon la revendication 12, **caractérisé en ce que** le corps creux (66b) présente un contour extérieur (66c) se réduisant vers le haut, et **en ce que** le corps creux (66b) est disposé de telle sorte dans le contenant (62) que le premier espace d'écoulement (66a) s'élargissant de bas en haut est réalisé entre le contour extérieur (66c) et la paroi de contenant (62d).

15. Bioréacteur (61) selon l'une des revendications 6 à 14, **caractérisé en ce que** le contenant (62) présente en haut au moins une ouverture pouvant être fermée (63, 63a, 63b, 63c).
